# EUROPEAN PATENT APPLICATION

(11) **EP 3 375 868 A1**
(43) Date of publication of application: **19.09.2018**
(21) Application number: 18150910.0
(22) Date of filing: 07.11.2008
(51) Int. Cl.: C12N 5/12, C12N 5/16, A61K 39/00, G01N 33/00

(54) **STIMULATION OF ANTI-TUMOR IMMUNITY USING DENDRITIC CELL/TUMOR CELL FUSIONS AND ANTI-CD3/CD28**

(30) Priority: 08.11.2007 US 2538 P
(62) Divisional of application: 08846621.4
(71) Applicant: Dana Farber Cancer Institute, Inc., Boston, Mssachusetts 02215 (US); Beth Israel Deaconess Medical Center, Boston, MA 02215 (US)
(72) Inventor: AVIGAN, David, Boston, MA 02115 (US); KUFE, Donald, Wellesley, MA 02482 (US)
(74) Representative: Helbig, Christian

(57) **Abstract**

The invention is concerned with fusions of dendritic cells and with tumor or cancer cells. Also provided are methods of making and using these cell fusions, including methods of adoptive immunotherapy as well as methods of stimulating anti-tumor immunity using fused cells and anti-CD3/CD28 antibodies.

## Description

### RELATED APPLICATIONS

This application claims the benefit of U.S.S.N. 61/002,538, filed November 8, 2007, which is incorporated herein by reference in its entirety.

### Statement as to Federally Sponsored Research

This invention was made with U.S. government support under Department of Defense Grant DAMD17-03-1-04R7; Renal SPORE Development Project Grant CA10194; and Ovarian Cancer SPORE Grant CA105009. The United States government has certain rights in this invention.

### Field of the Invention

The invention relates generally to cellular immunology.

### Background of the Invention

Tumor cells express unique antigens that are potentially recognized by the host T cell repertoire and serve as potential targets for tumor immunotherapy. However, tumor cells evade host immunity because antigen is presented in the absence of costimulation, and tumor cells express inhibitory cytokines that suppress native antigen presenting and effector cell populations. (*See* Speiser et al, J. Exp. Med. 186:645-53 (1997); Gabrilovich et al., Clin Cancer Res. 3:483-90 (1997)). A key element in this immunosuppressive milieu is the increased presence of regulatory T cells that are found in the tumor bed, draining lymph nodes, and circulation of patients with malignancy. *(See* von Boehmer, Nat Immunol 6:338-44 (2005); Liyanage et al., J. Immunol. 169:2756-61 (2002)). Thus, a promising area of investigation is the development of cancer vaccines to reverse tumor associated anergy and to stimulate effector cells to recognize and eliminate malignant cells.

### Summary of the Invention

The invention features compositions for stimulating an immune system. Accordingly, the invention includes a hybrid cell (or progeny thereof), which is a fusion product of a dendritic cell, *e.g.,* a non-follicular dendritic cell, and non-dendritic cell. The hybrid cell expresses B7 (*e*.*g*. any member of the B7 family of costimulatory molecules such as B7-1 or B7-2) on its surface. Preferably, the hybrid cell also expresses other costimulatory molecules, MHC class I and class II molecules, and adhesion molecules. The dendritic cell fusion partner and the non-dendritic cell may be derived from the same species. Examples include hybrid cells in which the non-dendritic cell fusion partner expresses a disease-associated antigen such as that derived from a tumor, a bacterium, or a virus. Alternatively, the non-dendritic cell is a tumor cell. The dendritic cell is autologous or allogeneic. The dendritic cell and the non-dendritic cell are preferably derived from the same individual, *e*.*g*., a human patient.

These immunostimulatory compositions each contain a plurality of cells containing fused cells, each of which fused cells is generated by fusion between at least one mammalian dendritic cell (*e.g.,* a DC derived from a bone marrow culture or a peripheral blood cell culture) and at least one mammalian non-dendritic cell (*e.g.,* a cancer cell or a transfected cell) that expresses a cell-surface antigen (*e.g.,* a cancer antigen). By "cancer antigen" is meant an antigenic molecule that is expressed primarily or entirely by cancer cells, as opposed to normal cells in an individual bearing the cancer. A cancer antigen may also be expressed at higher levels by a malignant cell as compared to its normal counterpart. Alternatively, a cancer antigen may be expressed specifically in certain malignant and normal cells (*i.e.,* prostate specific antigen). The fused cells within the compositions express, in an amount effective to stimulate an immune system (*e.g*., to activate T cells), MHC class II molecules, B7, and the cell-surface antigen.

This invention also provides a substantially pure population of educated, antigen-specific immune effector cells expanded in culture at the expense of hybrid cells, wherein the hybrid cells are antigen presenting cells (APCs) fused to cells that express one or more antigens. The invention also includes a population of activated and expanded immune effector cells. For example, the cells are activated *ex vivo.* The population contains T cells and hybrid cells. The cells can be derived from a coculture of a patient-derived immune cell and a hybrid cell. Effector cells specifically kill autologous tumor cells and recognize a known or unknown tumor antigen and can therefore be used to identify unknown tumor antigens.

Also provided herein are methods of producing substantially pure, educated, expanded, antigen-specific populations of immune effector cells, wherein the immune effector cells are T-lymphocytes and wherein the population contains both CD4⁺ immune effector cells and cytotoxic CD8⁺ immune effector cells. Specifically, such methods involve the steps of providing a plurality of hybrid cells, each of which hybrid cells is generated by fusion between at least one dendritic cell and at least one tumor or cancer cell that expresses a cell-surface antigen, wherein the dendritic cell and the tumor or cancer cell are from the same species, wherein the dendritic cell can process and present antigens, and wherein at least half of the hybrid cells express, in an amount effective to stimulate the immune system, (a) MHC class II molecule, (b) B7, and (c) the cell-surface antigen; contacting a population of immune effector cells with the plurality of hybrid cells, thereby producing a population of educated, antigen-specific immune effector cells; and contacting the resulting population with anti-CD3/CD28 antibody in order to increase T cell expansion, T cell activity, and/or tumor-reactive T cells, as compared to exposure to the hybrid cells or to the anti-CD3/CD28 antibody alone. For example, these methods may result in at least about a two-fold increase in activated T cells at least about a two-fold increase in tumor reactive T-cells; and/or at least about a two-fold increase in T-cell expansion. Increase in stimulation with DC/tumor fusions followed by anti-CD3/CD28 as compared to stimulation with DC/tumor fusions alone can be measured by examining one more characteristics, including, but not limited to, extent of T cell proliferation; presence of memory effector cells; increased presence of activated T cells within the population (*e*.*g*. by measuring CD69 expression); the presence of cells expressing IFNγ and/or granzyme B; the presence of tumor reactive T cells (*e.g.* by tetramer staining); and/or decreased presence of regulatory T cells within the population (*e.g.* by measuring FoxP3 expression).

Those skilled in the art will recognize that the methods of the invention result in an increased number of both activated and regulatory T cells. However, a greater percentage of activated T cell is observed when compared to the number of regulatory T cells observed following exposure to the fusions and expansion with the anti-CD3/CD28 antibody. Thus, the resulting T cell population primarily manifests an activated phenotype.

Optionally, the methods of the invention also include the step of contacting the educated, expanded T cell population with compound(s) that remove or otherwise decrease the activity of regulatory T cells following expansion with the anti-CD3/CD28 antibody. Compounds that remove or decrease the activity of regulatory T cells include, for example, certain cytokines. It is also possible that the activity of regulatory T cells can be accomplished by the use of selection methods or by silencing of key genes in regulatory T cells by using siRNAs.

Those skilled in the art will recognize that the anti-CD3/CD28 antibody is bound to a flat substrate or to any other suitable substrate or surface commonly used in the art such that the immune effector cells can be expanded in at least 24 hours.

In accordance with these methods, the immune effector cells and/or the hybrid cells may be genetically modified cells. For example, the genetic modification may involve the introduction of a polynucleotide encoding a peptide, a ribozyme, an antisense sequence, a hormone, an enzyme, a growth factor, and/or an interferon into the cell(s).

Additionally, the immune effector cells may be naive prior to culturing with the hybrid cells. Moreover, the immune effector cells may be cultured with the hybrid cells in the presence of one or more cytokines or adjuvants. Suitable cytokines include, but are not limited to IL-7, IL-12 and/or IL-18. Moreover, suitable adjuvants may include, but are not limited to CPG ODN, a TLR7/8 agonist, and/or a TLR3 agonist.

The resulting expanded, educated, antigen-specific population of immune effector cells can be maintained in a cell culture medium comprising a cytokine such as IL-7.

Those skilled in the art will recognize that the dendritic cell and the tumor or cancer cell that expresses one or more antigens may be autologous or allogeneic. In some embodiments, the dendritic cell and the tumor or cancer cell are obtained from the same individual (*i.e.* from the same human). Alternatively, the dendritic cell and the tumor or cancer cell are obtained from different individuals of the same species (*i.e., Homo sapiens*).

Suitable dendritic cells for use in these methods may be derived or obtained from peripheral blood, bone marrow or skin. Likewise, the dendritic cell can be obtained or derived from a dendritic cell progenitor cell.

The tumor or cancer cells contemplated for use in connection with these methods include, but are not limited to, breast cancer cells, ovarian cancer cells, pancreatic cancer cells, prostate gland cancer cells, renal cancer cells, lung cancer cells, urothelial cancer cells, colon cancer cells, rectal cancer cells, or hematological cancer cells. For example, hematological cancer cells include, but are not limited to, acute myeloid leukemia cells, acute lymphoid leukemia cells, multiple myeloma cells, and non-Hodgkin's lymphoma cells.

Moreover, those skilled in the art would recognize that any tumor or cancer cell may be used in any of the methods of the present invention.

Also provided herein are substantially pure populations including expanded, educated, antigen-specific immune effector cells, wherein the population comprises educated, antigen-specific immune effector cells that are educated by hybrid cells that include dendritic cells fused to tumor or cancer cells that express one or more antigens. Preferably, the dendritic cell and the tumor or cancer cell are from the same species, the dendritic cell can process and present antigens, and at least half of the fused cells express, in an amount effective to stimulate the immune system, (a) a MHC class II molecule, (b) B7, and (c) the cell-surface antigen. The resulting educated, immune effector cells are subsequently expanded in culture in the presence of anti-CD3/CD28 antibody, wherein following this expansion in culture, T cell expansion in the population is at least about seven-fold increased, T-cell activation in the population is at least about four fold increased, tumor-reactive T-cells in the population are at least about thirteen fold increased, or any combination thereof, as compared to immune effector cells exposed to the hybrid cells alone.

The dendritic cell and the tumor or cancer cell are obtained from the same individual (i.e., the same human) or from different individuals of the same species (*i.e., Homo sapiens*).

In one example, it has been observed that, when the tumor or cancer cell is a renal carcinoma cell, T cell proliferation in the population is at least about thirteen fold increased as compared to immune effector cells exposed to the hybrid cells alone; the presence of memory effector cells in the population is at least about two fold increased as compared to immune effector cells exposed to hybrid cells alone T cell activation in the population is at least about eight fold increased as compared to immune effector cells exposed to the hybrid cells alone; the presence of cells expressing IFNγ and granzyme B in the population is increased at least about 2.5 fold and 3.75 fold, respectively, as compared to immune effector cells exposed to the hybrid cells alone; and tumor reactive T cells in the population are at least about thirteen fold increased as compared to immune effector cells exposed to the hybrid cells alone, following expansion in culture in the presence of anti-CD3/CD28 antibody.

Those skilled in the art would recognize that the fold increase in numbers of various cells in the population would depend on the type of tumor or cancer cell used in the present invention. In addition, there is patient to patient variability within a particular cancer type.

The mean fold increase of stimulation with DC/tumor fusions followed by anti-CD3/CD28 as compared to stimulation with DC/tumor fusions alone is shown below in Table 1.

**Table 1.**

| | **Proliferation** | **Memory 45RO** | **CD69** | **IFN** | **FoxP3** | **Granzyme** | **Tetramer** | |
|---|---|---|---|---|---|---|---|---|
| **Renal** | 13.2 | 2 | 8 | 2.5 | 7.5 | 3.75 | 8.5 | |
| **AML** | 2.5 | 4 | | | | 5.2 | | |
| **Breast** | 7.4 | | 5 | 4 | 5 | | 13.7 | |

The resulting population of expanded, educated, antigen-specific immune effector cells can also be used as a vaccine that may contain the population of cells and a pharmaceutically acceptable carrier.

Also provided herein are methods of treating cancer by administering this population of expanded, educated immune effector cells to an individual in order to induce an immune response. For example, the cancer to be treated is selected from the group consisting of breast cancer, ovarian cancer, pancreatic cancer, prostate gland cancer, renal cancer, lung cancer, urothelial cancer, colon cancer, rectal cancer, brain cancer (*e.g*., glioma), or hematological cancer. For example, suitable hematological cancers may include, but are not limited to, acute myeloid leukemia, acute lymphoid leukemia, multiple myeloma, and non-Hodgkin's lymphoma.

Those skilled in the art will recognize that such treatment methods may also involve the co-administration of an effective amount of a plurality of hybrid cells, each of which hybrid cells is generated by fusion between at least one dendritic cell and at least one tumor or cancer cell that expresses a cell-surface antigen, wherein the dendritic cell and the tumor or cancer cells are from the same species, and wherein at least half of the hybrid cells express, in an amount effective to stimulate the immune system, (a) MHC class II molecule, (b) B7, and (c) the cell-surface antigen. For example, the co-administration may occurs sequentially or simultaneously.

In addition, the individual in need of treatment may be given a treatment to deplete lymphocytes prior to administration of the population. Specifically, this treatment induces lymphopenia in the individual. Examples of suitable treatments include, but are not limited to, the administration of fludarabine or radiation.

The population of expanded, educated immune effector cells may be administered to the individual subsequent to stem cell transplantation.

The invention also features methods of testing peptides for antigenic activity. Specifically, such methods include the steps of providing a hybrid cell including a fusion product of a dendritic cell and a tumor or cancer cell, wherein the hybrid cell expresses B7 on its surface; contacting the hybrid cell with an immune effector cell, thereby producing an educated immune effector cell; contacting the educated immune effector cell with an anti-CD3/CD28 antibody; and contacting a target cell with the educated immune effector cell in the presence of a peptide. Those skilled in the art will recognize that lysis of the target cell identifies the peptide as an antigenic peptide.

Also provided are methods of testing a peptide for antigenic activity involve the steps of providing a plurality of cells, wherein at least 5% of the plurality of cells are fused cells generated by fusion between at least one dendritic cell and at least one tumor or cancer cell that expresses a cell-surface antigen, wherein the fused cells express, in amounts effective to stimulate an immune response, (a) MHC class II molecule, (ii) B7, and (iii) the cell-surface antigen; contacting a population of human T lymphocytes with the plurality of cells, wherein the contacting causes differentiation of effector cell precursor cells in the population of T lymphocytes to effector cells comprising cytotoxic T lymphocytes; contacting the effector cells comprising cytotoxic T lymphocytes with an anti-CD3/CD28 antibody; and contacting a plurality of target cells with the effector cells comprising T lymphocytes in the presence of the peptide. In such methods, lysis of the plurality of target cells or a portion thereof identifies the peptide as an antigenic peptide that is recognized by the cytotoxic T lymphocytes.

Finally, the invention also provides vaccines containing an antigenic peptide identified according to any of the methods disclosed herein and a carrier.

Unless otherwise defined, all technical and scientific terms used herein have the same meaning as commonly understood by one of ordinary skill in the art to which this invention belongs. All citations herein are incorporated by reference in their entirety.

Other features and advantages of the invention will be apparent from the following drawings, detailed description, and from the claims.

### Brief Description of the Drawings

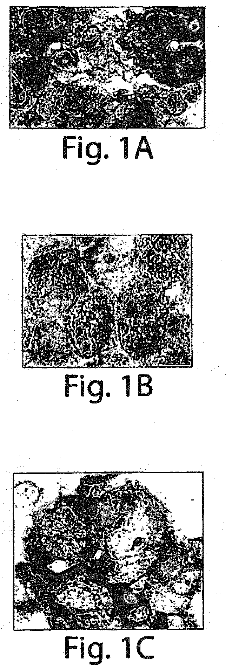

Figures 1A-1C show the results of immunohistochemical analysis of monocyte derived dendritic cells (DCs), the renal cell carcinoma ("RCC") cell line, RCC 786, and fusion cells. DCs were generated from adherent mononuclear cells isolated from leukopak collections obtained from normal donors. DCs were cultured with GM-CSF and IL-4 for 5 days and then underwent maturation by exposure to TNFα for 48-72 hours. DC preparations underwent immunohistochemical analysis for expression of costimulatory molecules. DC expression of CD86 (blue) is shown (60X) in Figure 1A. RCC 786 cells were cultured in RPMI 1640 complete medium and underwent immunohistochemical analysis for expression of the tumor associated antigens cytokeratin and CAM. Tumor expression of CAM (red) is shown (60X) in Figure 1B. Fusion cells were generated by co-culture of DCs and RCC 786 cells in the presence of PEG. Fusion cell preparations underwent immunohistochemical analysis for co-expression of the DC derived costimulatory molecule CD86 (blue) and tumor associated antigen CAM (red) (Figure 1C).

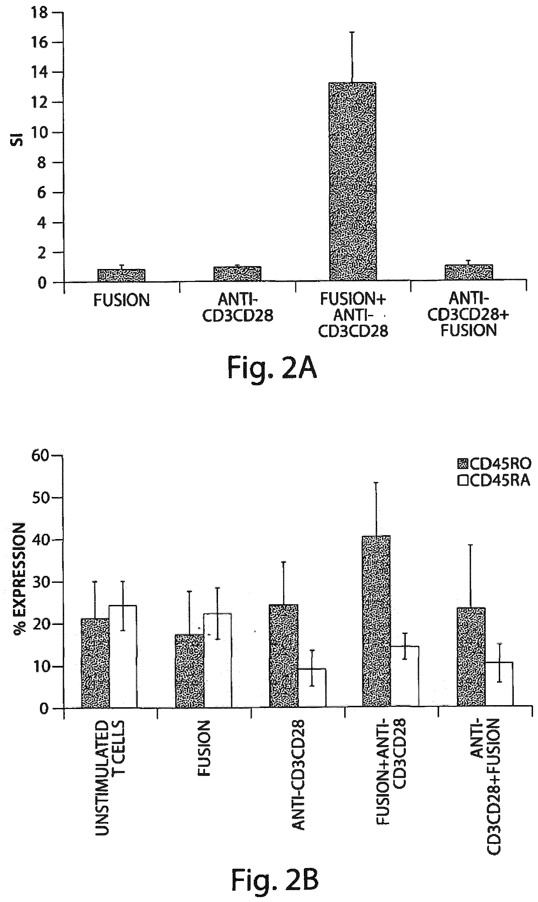

Figures 2A-2B show the effect of stimulation by fusion cells, anti-CD3/CD28, or sequential stimulation with fusions and anti-CD3/CD28 on T cell proliferation. T cells were: 1) cocultured with fusion cells for 7 days at a fusion to T cell ratio of 1:10; 2) cultured on the anti-CD3/CD28 coated plates for 48 h; 3) cocultured with fusion cells for 5 days followed by anti-CD3/CD28 coated plates for 48 h; or 4) cultured with anti-CD3/CD28 for 48 h followed by stimulation with fusion cells for 5 days. Following stimulation, T cell proliferation was measured by uptake of tritiated thymidine following an overnight pulse. Figure 2A shows the results expressed as a stimulation index (T cell proliferation following coculture/Proliferation of unstimulated T cells). Mean values of 9 experiments, with associated standard error of the means are presented. T cells stimulated by fusion cells, anti-CD3/CD28, or sequential stimulation with fusions and anti-CD3/CD28 underwent phenotypic analysis to assess for the presence of naive (CD45 RA) and memory (CD45RO) T cell populations. Stimulated T cells were incubated with FITC conjugated CD4 and PE conjugated CD45RA or CD45RO and analyzed by flow cytometry. Mean values of 4 experiments with associated standard error of the means are shown in Figure 2B.

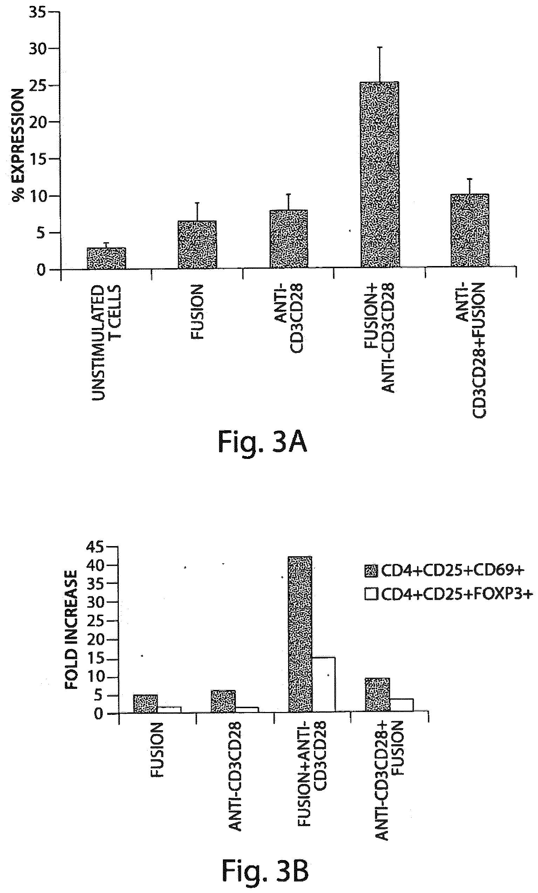

Figure 3 shows the results of phenotype analysis of T cells stimulated by fusion cells, anti-CD3/CD28, or sequential stimulation with fusions and anti-CD3/CD28. T cells stimulated by fusion cells, anti-CD3/CD28, or sequential stimulation with fusions and anti-CD3/CD28 underwent phenotypic analysis by multichannel flow cytometry to assess for co-expression of CD4 and CD25. Figure 3A shows the results of stimulated T cell populations stained with FITC conjugated CD4 and cychrome conjugated CD25 to determine the percentage of dually expressing cells. Mean values of 11 experiments are presented with associated standard error of the means. Whether combined stimulation with DC/RCC fusions and anti-CD3/CD28 results in the expansion of activated (CD4+CD25+CD69+) as compared to regulatory T cells (CD4+CD25+FOXP3+) was examined. Stimulated T cell preparations were stained for FITC conjugated CD4, cychrome conjugated CD25, and PE conjugated CD69. Alternatively, cells were stained for CD4/CD25, permeabilized, and incubated with PE conjugated Foxp3 or a matched isotype control antibody. CD4/CD25+ T cells were isolated by FACS gating and expression of CD69 and Foxp3 was determined. As shown in Figure 3B, results are presented as the percentage of activated or regulatory T cells out of the total T cell population. Mean values of 9 experiments with associated standard error of the means are presented.

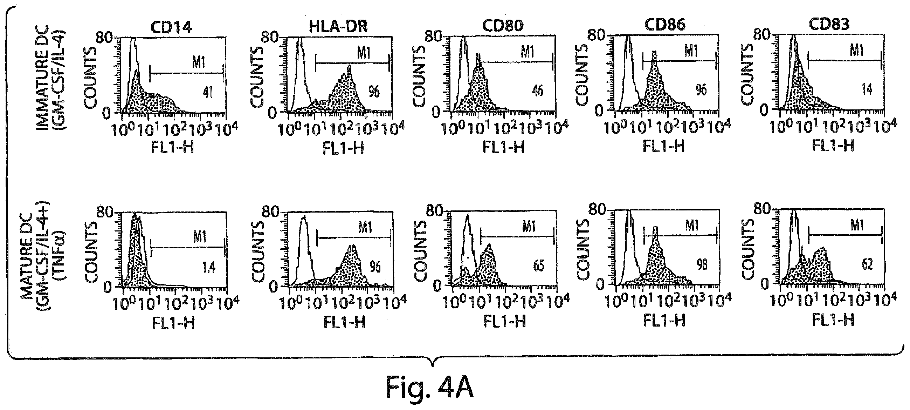

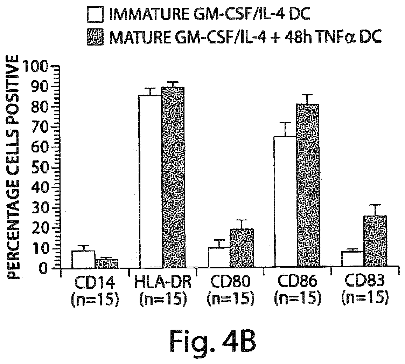

Figure 4 shows the results of phenotype analysis of monocyte derived dendritic cells (DCs). DCs were generated from adherent mononuclear cell isolated from peripheral blood of breast cancer patients and leukopaks obtained from normal donors. Cells were cultured with GM-CSF (1000 IU/ml) and IL-4 (1000 IU/ml) for 5-7 days (immature DCs) and a subset underwent maturation with TNFα (25 nglml) for 48-72 hours. Immature and mature DCs underwent FACS analysis to assess expression of costimulatory and maturation markers. Figure 4A shows the FACs analysis of a representative immature and mature DC preparation. Figure 4B shows the mean percentage (± SEM) of cells expressing the indicated surface marker for 15 experiments. Maturation results in increased expression of costimulatory (CD80 and CD86) and maturation (CD83) markers.

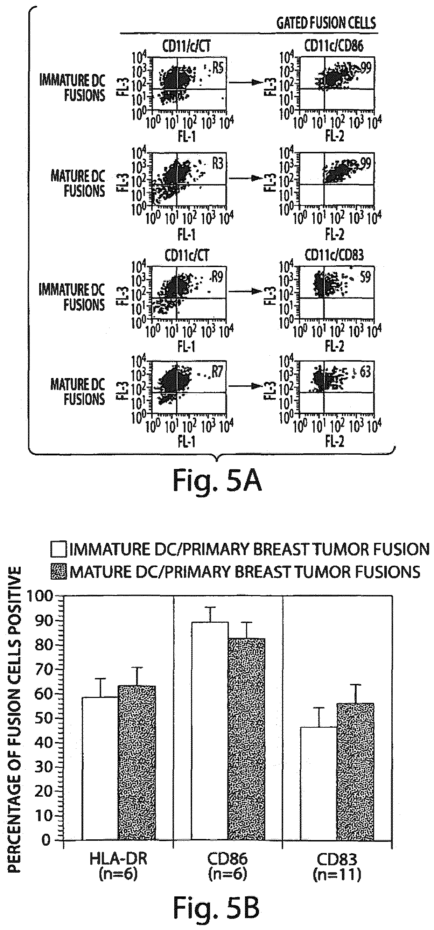

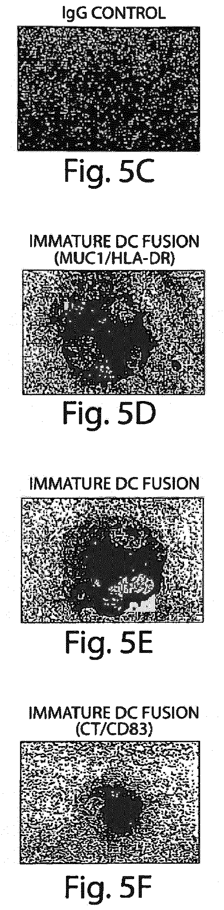

Figure 5 shows the results of phenotypic analysis of DC/breast carcincoma fusion cells. Tumor cells were fused with immature or mature DCs by coculture in the presence of PEG. Figure 5A shows the results of a representative experiment, where fusion cells were isolated by gating around cells that coexpressed cytokeratin (CT) and CD11c (left panel). Expression of CD86 and CD83 by the fusion cells was determined (right panel). Figure 5B shows the mean percentage (± SEM) of immature and mature DC/breast carcinoma fusions expressing DR, CD86, and CD83. Immunohistochemical analysis of DC-tumor fusion preparations was performed following cytospin preparation. Immature DC/breast carcinoma fusions were stained for isotype matched IgG control (Figure 5C); MUC1/HLA-DR (Figure 5D); CT/CD86 (Figure 5E); and CT/CD83 (Figure 5F).

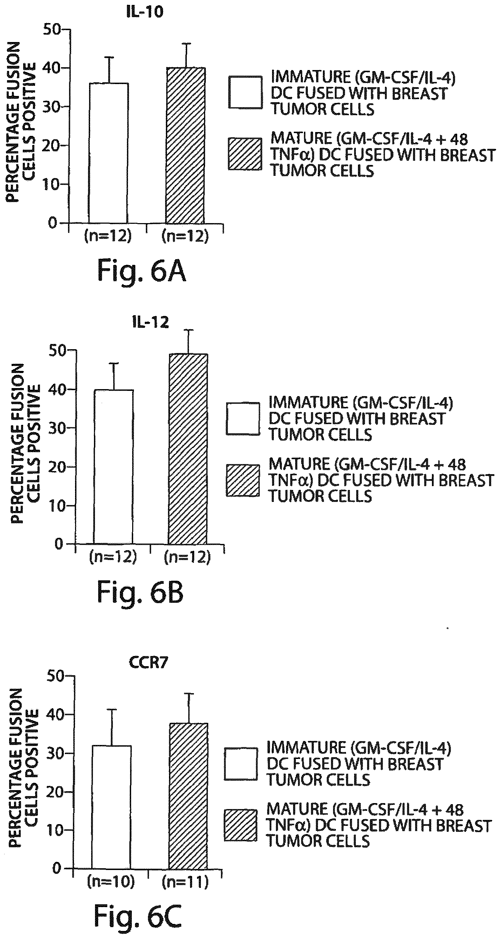

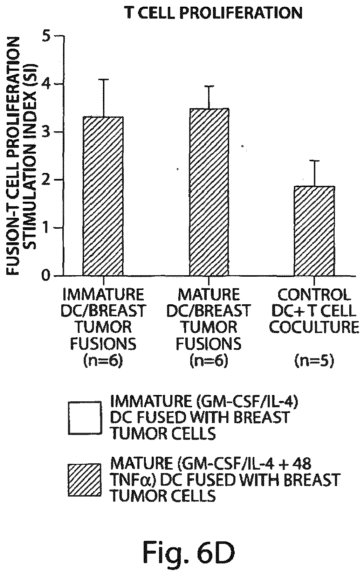

Figure 6 shows the expression of IL-10, IL-12, and CCR7 in DC/breast carcinoma fusion cells generated with either immature or mature DCs. Fusion cell preparations generated with immature or mature DCs were stained with CT and CD11c and subsequently fixed, permeabilized and stained for intracellular IL-10 and IL-12. Unfixed fusion cells were used for the surface expression of CCR7. Fusion cells were isolated by FACS gating and analyzed for expression of IL-10, IL-12, and CCR7. The mean percentage (± SEM) of immature and mature DC/breast carcinoma cells expressing IL-10 (Figure 6A); IL-12 (Figure 6B); and CCR7 (Figure 6C) is shown for 12 experiments. Figure 6D shows the induction of T cell proliferation by DC/breast carcinoma cells prepared with immature or mature DCs. Fusion cells were cocultured with T cells and proliferation was measured by ³[H]=Thymidine uptake. Results were normalized by calculation of stimulation index (SI).

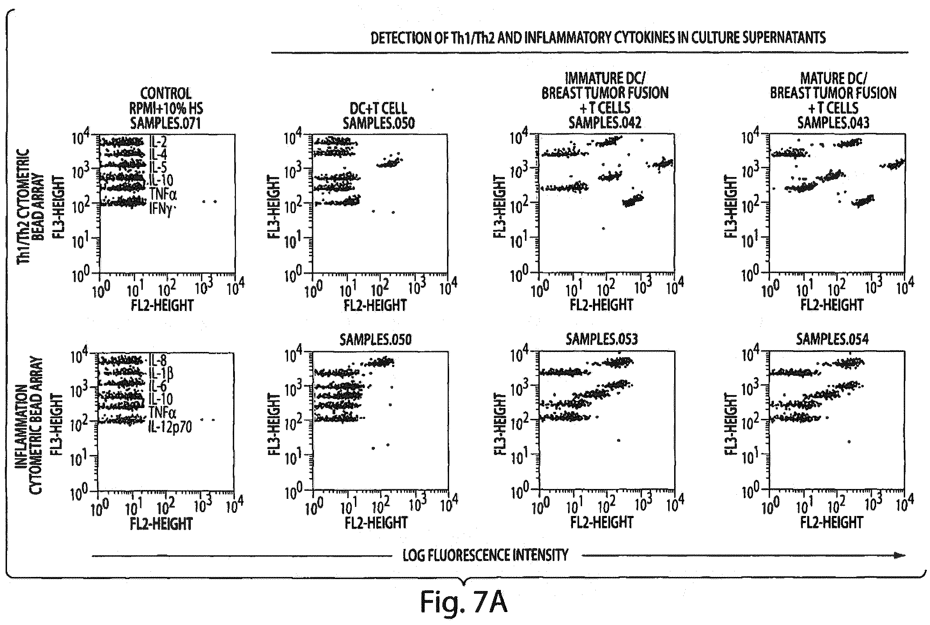

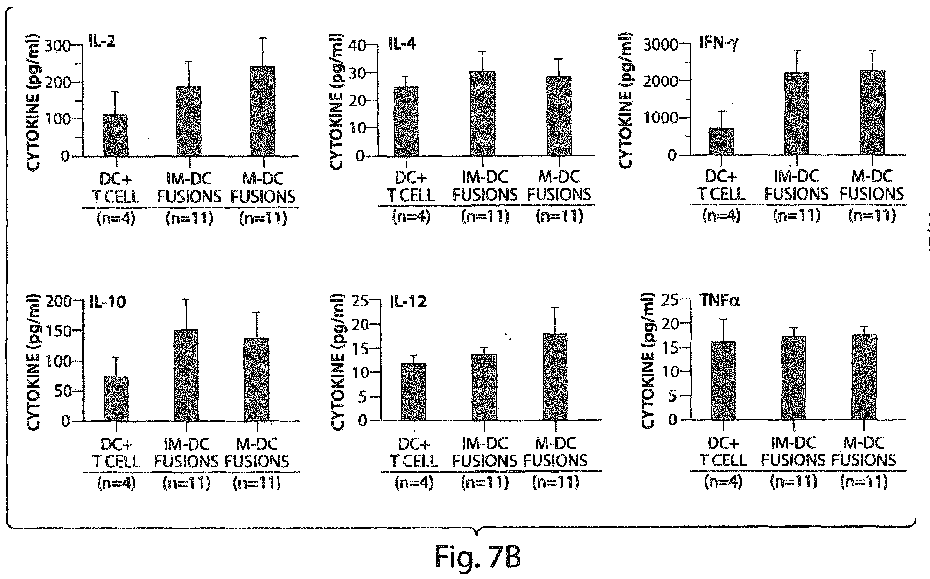

Figure 7 shows the culture supernatant expression of cytokines following autologous T cell stimulation with DC/breast carcinoma fusions. The Th1, Th2, and inflammatory cytokine profiles of culture supernatants of immature and mature DC/breast carcinoma fusion cells cocultured with autologous non-adherent cells were quantitated using the cytometric bead array (CBA) analysis kit. In Figure 7A, the upper panel shows a representative example from a single experiment depicting the fluorescence bead array dot-plot assay display for Th1/Th2 and inflammatory cytokines after data acquisition with BD CellQuest software followed by data formatting and subsequent analysis using the BD CBA software. In Figure 7B, the mean (± SEM) concentration of IL-2, IL-4, IL-10, IL-12, TNFα, and IFNγ cytokine (pg/ml) in culture supernatants is presented from a series of 4 (DCs + autologous non-adherent cell cocultures as controls) and 11 (immature and mature DC/breast carcinoma fusion cells cocultured with autologous nonadherent cells) separate experiments. (IM-DC fusions: immature dendritic cell fusions; M-DC fusions: mature dendritic cell fusions).

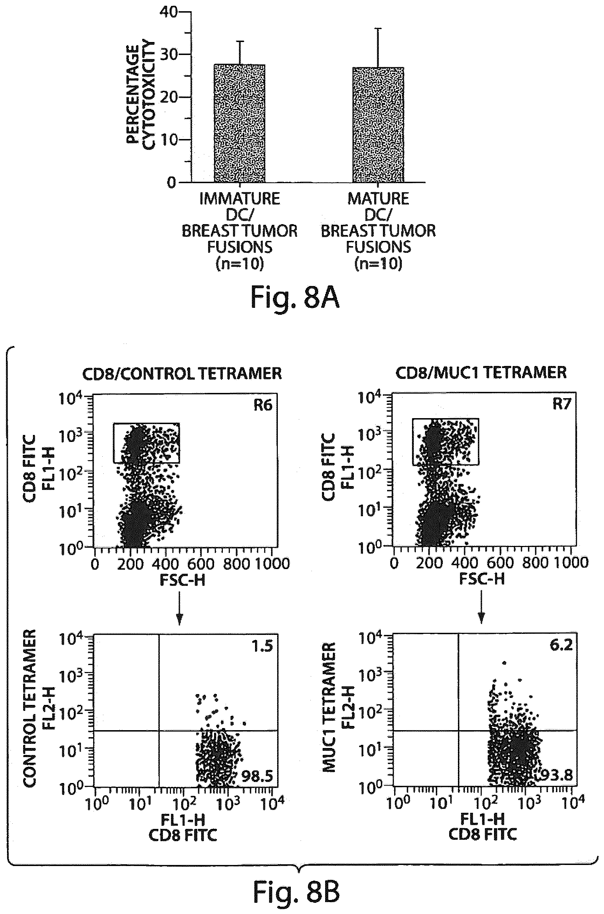

Figure 8 shows that immature and mature DC/breast carcinoma fusions stimulate lysis of tumor targets and expansion of MUC-1 specific T cells. In Figure 8A, immature and mature DC/breast carcinoma fusion cells were cocultured with autologous T cells at a ratio of 30:1 for 7-10 days. T cells were incubated with ⁵¹Cr labeled autologous breast tumor cells or semi-autologous DC/breast carcinoma fusion cells. Lysis of the labeled cells was determined by chromium release assay. The mean percentage cytotoxicity (± SEM) following stimulation with immature or mature DC/breast carcinoma fusion cells is presented. Figure 8B shows that stimulation with mature DC/breast carcinoma fusions results in the expansion of T cells binding the MUC1 tetramer. DCs generated from HLA*0201 donors were fused with breast carcinoma cells and cultured with autologous T cells for 5 days. The percent of CD8+ T cells binding the MUC1 tetramer prior to and following fusion cell stimulation was determined by bidimensional FACS analysis and compared to that seen with a control tetramer.

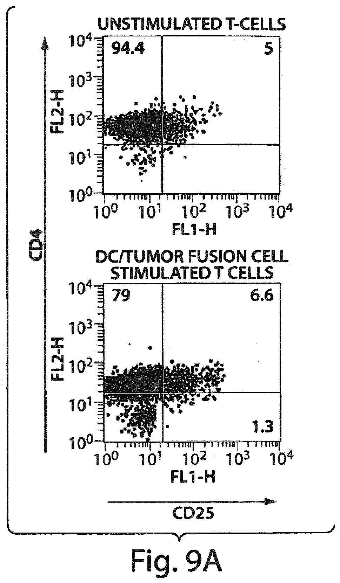

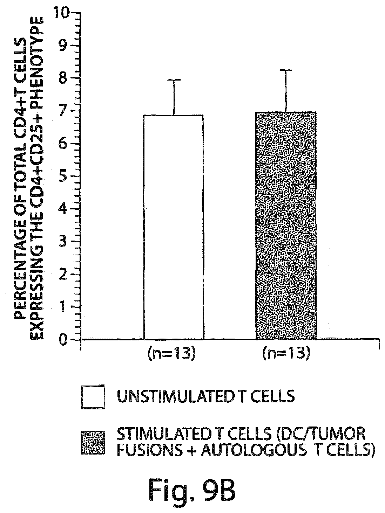

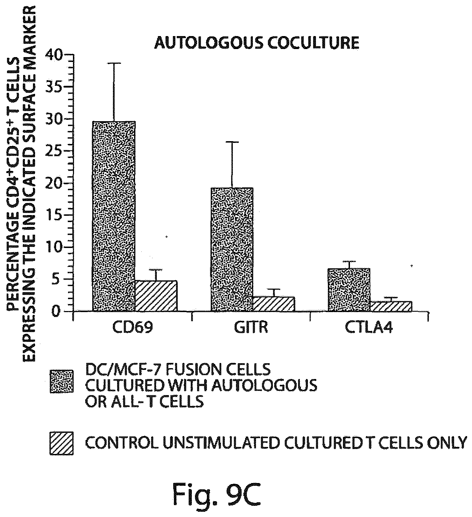

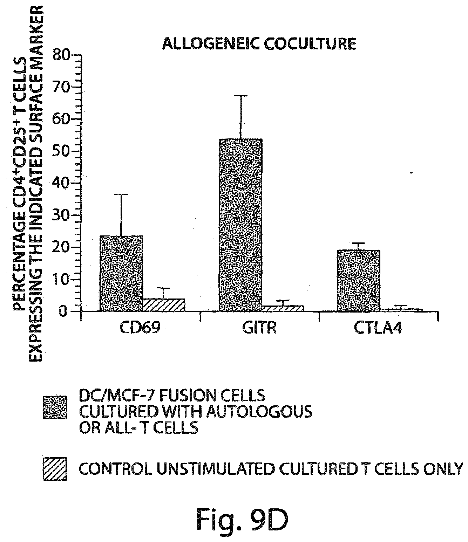

Figure 9 shows that stimulation with DC/breast carcinoma fusions results in the expansion of activated and regulatory T cells. In Figure 9A, autologous non-adherent T cells were stimulated with DC/breast carcinoma fusion cells for 5 days. CD4+ T cells were selected using magnetic microbeads (Miltenyi Biotec) and labeled with PE-conjugated CD4 and FITC-conjugated CD25 antibodies. CD4+CD25+ cells were quantified by a bidimensional FACS analysis for unstimulated and fusion stimulated T cells. Data is presented from a representative dot plot experiment. Figure 9B shows the mean percentage (± SEM) of CD4+CD25+ T cells. Autologous (Figure 9C) or allogeneic (Figure 9D) T cells were cultured with DC/breast carcinoma fusion cell for 5 days and CD4+ T cells were isolated by magnetic bead separation. The mean percentage (± SEM) of cells that coexpressed CD25/CD69, CD25/CITR, and CD25/CTLA-4 was determined by bidimensional flow cytometry. Data is representative (mean ± SEM) of 5 separate experiments.

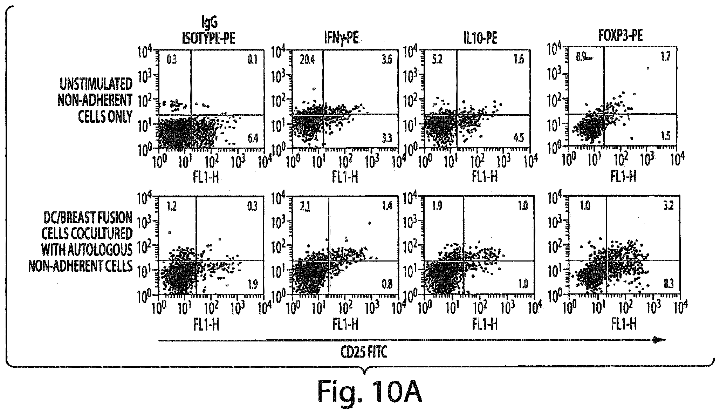

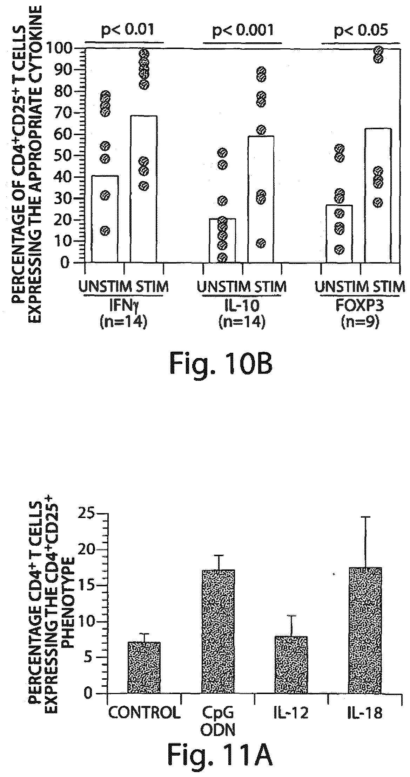

Figure 10 shows the expansion of T cells following IFNγ, IL-10, and Foxp3 following stimulation with DC/breast carcinoma fusion cells. Autologous T cells were cocultured with DC/breast carcinoma fusions for 5-7 days. Following selection of CD4+ T cells using magnetic microbeads, cells were stained with FITC conjugated CD25, permeabilized with Cytofix/Cytoperm solution, and stained with PE-conjugated IFNγ, IL-10 or Foxp3 antibodies. Figure 10A shows a representative FACS analysis of unstimulated (upper panel) and fusion stimulated CD4+CD25+ T cells (lower panel) expressing IFNγ, IL-10 or Foxp3. Figure 10B shows a stacking dot plot graph for a series of 9-14 experiments. The shaded histogram overlaying each dot plot group of experiments represents the mean for that group.

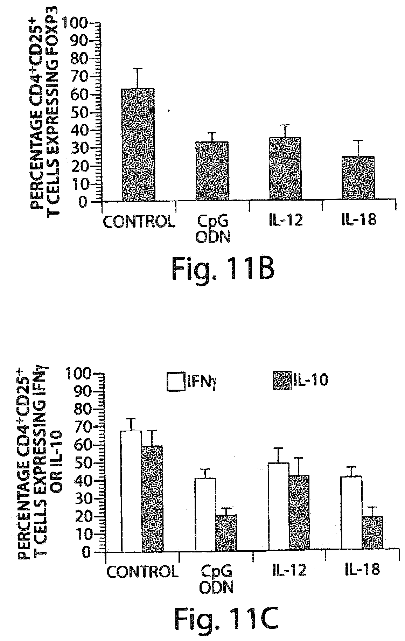

Figure 11 shows that the addition of CPG-ODN, IL12, and IL18 results in decreased expansion of regulatory T cells by DC/breast carcinoma fusions. DC/breast carcinoma fusion cells were cocultured with autologous T cells in the presence or absence of CpG ODN, IL-12, or IL-18 for a period of 5 days. Figure 11A shows that following selection of CD4+ cells, the percentage of CD4+/CD25+ was determined by bidimensional FACS analysis for each of the conditions. Figure 11B shows the mean percentage (± SEM) of CD4+CD25+ T cells expressing Foxp3 for each of the conditions determined by intracellular FACS analysis. Figure 11C shows the mean percentage (± SEM) of CD4+CD25+ T cells expressing IFNγ and IL-10 for each of the conditions determined by intracellular FACS analysis.

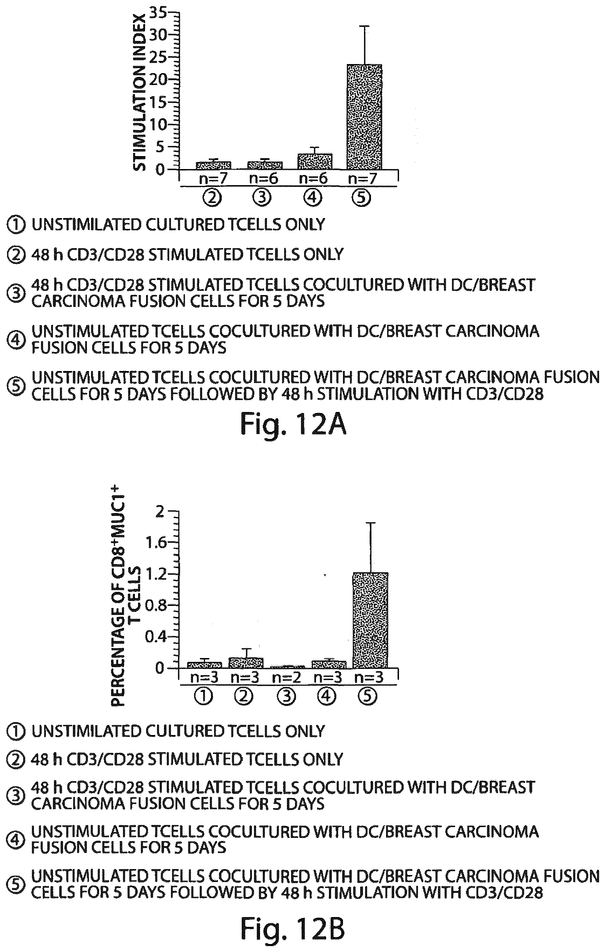

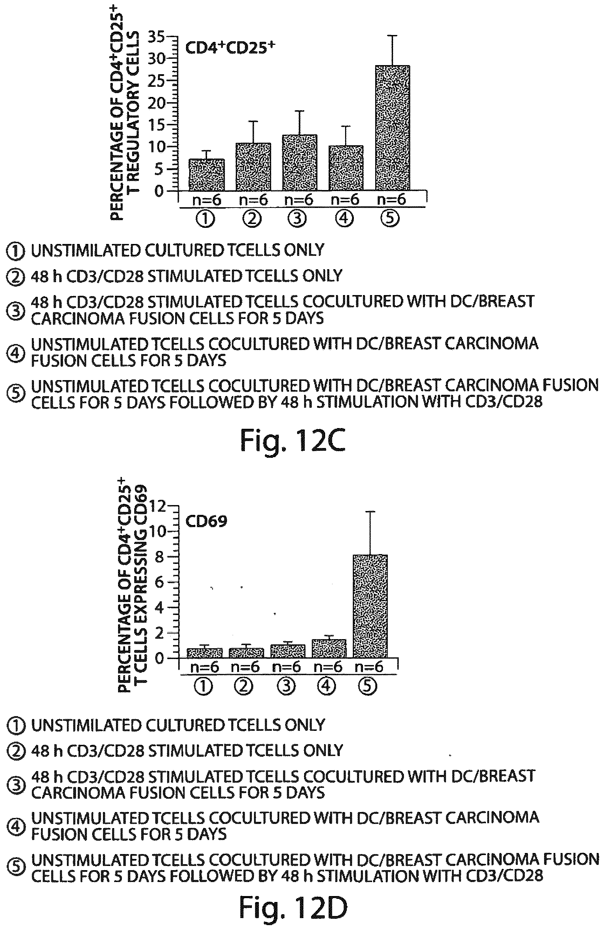

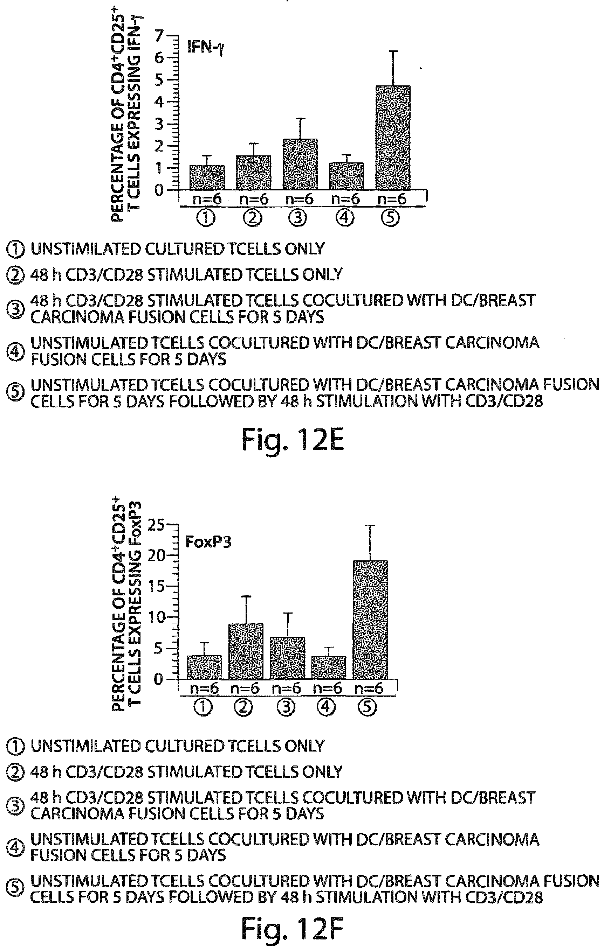

Figure 12 shows the results of combined stimulation with DC/breast carcinoma fusion cells and CD3/CD28 ligation. Autologous T cells were stimulated by culture with: DC/breast carcinoma fusion cells for 5 days; anti-CD3/CD28 coated plates for 48 hours; anti-CD3/CD28 followed by DC/breast carcinoma fusions; or DC/breast carcinoma fusions followed by anti-CD3/CD28. Results were compared to unstimulated T cells. Figure 12A shows the mean T cell proliferation for all culture conditions (n=6-7). T cells were aliquoted at 1x10⁵/well in triplicate in 96 well tissue culture plate and pulsed with 1 uCi/ml of ³[H]-Thymidine for a period of 18-24h. Results were normalized by calculation of stimulation index (SI). Mean expression of CD8+MUC1+ T cells using PE-conjugated MUC1 specific tetramers (Figure 12B); CD4+CD25+ T cells (n=6) (Figure 12C); CD4+CD25+CD69+ T cells (n=6) (Figure 12D); IFNγ expressing CD4+CD25+ T cells (n=5) (Figure 12E); and Foxp3 expressing CD4+CD25+ T cells (Figure 12F) is presented for each of the culture conditions listed.

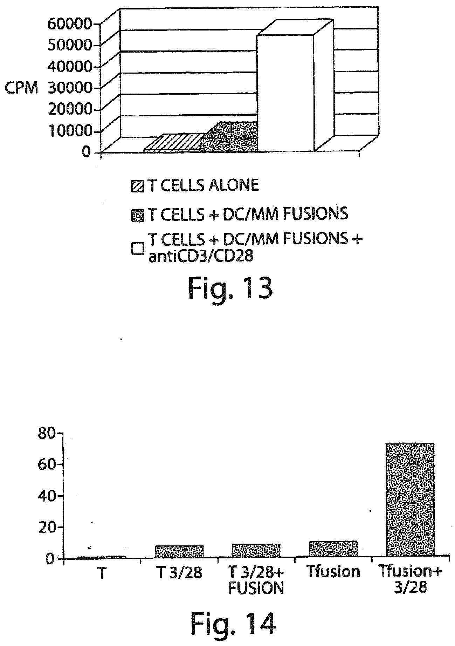

Figure 13 shows the effect of stimulation by DC/myeloma fusion cells or sequential stimulation with fusions and anti-CD3/CD28 on T cell proliferation. T cells derived from a patient with multiple myeloma (MM) were cocultured with fusion cells for 7 days at a fusion to T cell ratio of 1:10, or cocultured with fusion cells for 5 days followed by anti-CD3/CD28 coated plates for 48 h. Following stimulation, T cell proliferation was measured by uptake of tritiated thymidine following an overnight pulse.
Figure 14 shows the effect of autologous T cells stimulated by DC/myeloma fusion cells or sequentially by fusions and anti-CD3/CD28 on lysis of autologous tumor target cells. DC, tumor, and T cells were derived from a patient with multiple myeloma. Autologous T cells were either stimulated by anti-CD3CD28 alone for 48 hours, anti-CD3CD28 for 48 hours followed by DC/MM fusion stimulation for 5 days, DC/MM fusion cells alone for 7 days, or by DC/MM fusion cells for 5 days followed by exposure to anti-CD3CD28 for 48 hours. Figure 14 shows the percent lysis of autologous tumor target as determined in a standard ⁵¹Cr release assay.

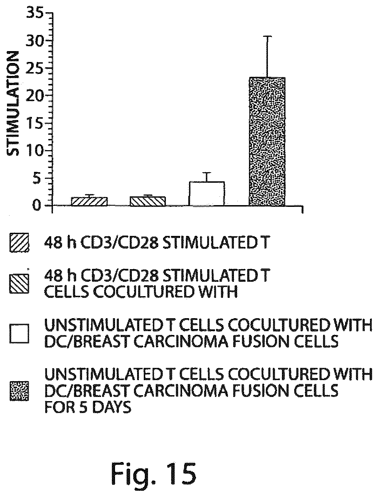

Figure 15 shows the mean T cell proliferation after stimulation with DC/breast carcinoma fusion cells and anti-CD3/CD28.

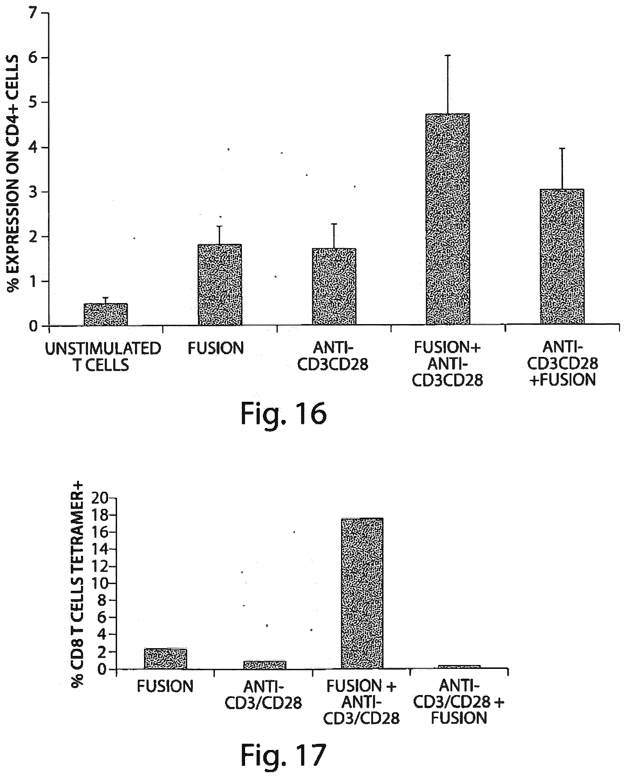

Figure 16 shows intracellular expression of IFNγ. Stimulated T cell preparations were stained for FITC conjugated CD4. Cells were then washed, permeabilized, and incubated with PE conjugated anti-human IFNγ or a matched isotype control antibody. Intracellular expression of IFNγ was determined by flow cytometric analysis. Mean values of 8 experiments are presented, with associated standard error of the means.
Figure 17 shows the percent CD8+ cells binding the MUC1 tetramer. HLA*0201+ autologous nonadherent cells were co-cultured with fusion cells, anti-CD3CD28, fusions followed by anti-CD3CD28 followed by fusion cells, and anti-CD3/CD28 followed by fusions cells. The cells were harvested and analyzed for MUC1+CD8+ T cells using the MUC1 speciflc PE-conjugated tetramers or a control tetramer and using the appropriate CD8+ T cell gating. The percent CD8+ cells binding the MUC1 tetramer (after subtraction of nonspecific binding to a control tetramer) is presented. Mean values from 2 experiments are presented.

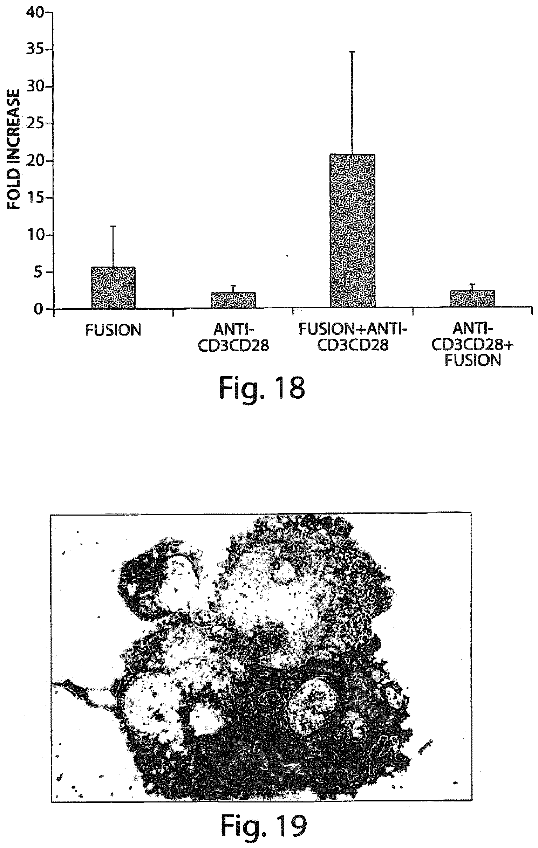

Figure 18 shows the percentage of CD8+ cells positive expressing granzyme B. T cells were cocultured with fusion cells, anti-CD3/CD28, fusion cells followed by anti-CD3/CD28, and anti-CD3CD28 followed by fusion cells. Cells were stained with FITC conjugated CD8 antibodies, fixed and permeabilized, incubated with PE-conjugated granzyme B antibody or matching isotype control and analyzed by flow cytometry. Bar graph shows the mean fold increase (± SEM) in the percentage of CD8+ cells positive expressing granzyme B.
Figure 19 shows immunohistochemical staining of the fusion cells. Myeloid leukemia cells were isolated from bone marrow aspirates or peripheral blood collections of patients with acute myeloid leukemia. Leukemia cells were fused with mature DCs using PEG. Fusion cells demonstrate co-expression of the tumor marker CD 117 (blue) and DC marker CD11C (red) by immunocytochemical staining (100x).

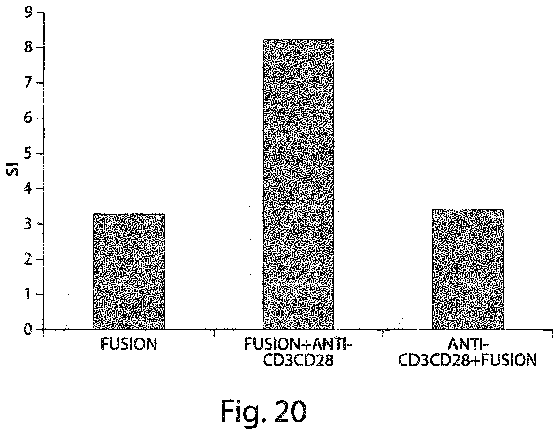

Figure 20 shows T cell proliferation (as measured by stimulation index) for T cells stimulated with DC/AML fusions, DC/AML fusions followed by anti-CD3/CD28, and anti-CD3/CD28 followed by DC/AML fusions.

### Detailed Description of the Invention

Various publications, patents and published patent specifications are referenced within the specification by an identifying citation. The disclosures of these publications, patents and published patent specifications are hereby incorporated by reference into the present disclosure to more fully describe the state of the art to which this invention pertains.

### Definitions

The practice of the present invention employs, unless otherwise indicated, conventional techniques of molecular biology, microbiology, cell biology and recombinant DNA, which are within the skill of the *art. See, e.g.,* Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel et al. eds., (1987)); the series METHODS IN ENZYMOLOGY (Academic Press, Inc.): PCR 2: A PRACTICAL APPROACH (Mi. MacPherson, B.D. Hames and G.R. Taylor eds. (1995)) and ANIMAL CELL CULTURE (Rd. Freshney, ed. (1987)).

As used herein, certain terms have the following defined meanings. As used in the specification and claims, the singular form "a", "an" and "the" include plural references unless the context clearly dictates otherwise. For example, the term "a cell" includes a plurality of cells, including mixtures thereof.

The term "immune effector cells" refers to cells that specifically recognize an antigen present, for example on a neoplastic or tumor cell. For the purposes of this invention, immune effector cells include, but are not limited to, B cells; monocytes; macrophages; NK cells; and T cells such as cytotoxic T lymphocytes (CTLs), for example CTL lines, CTL clones, and CTLs from tumor, inflammatory sites or other infiltrates. "T-lymphocytes" denotes lymphocytes that are phenotypically CD3+, typically detected using an anti-CD3 monoclonal antibody in combination with a suitable labeling technique. The T-lymphocytes of this invention are also generally positive for CD4, CD8, or both. The term "naïve" immune effector cells refers to immune effector cells that have not encountered antigen and is intended to by synonymous with unprimed and virgin. "Educated" refers to immune effector cells that have interacted with an antigen such that they differentiate into an antigen-specific cell.

The terms "antigen presenting cells" or "APCs" includes both intact, whole cells as well as other molecules which are capable of inducing the presentation of one or more antigens, preferably with class I MHC molecules. Examples of suitable APCs are discussed in detail below and include, but are not limited to, whole cells such as macrophages, dendritic cells, B cells; purified MHC class I molecules complexed to β2-microglobulin; and foster antigen presenting cells.

Dendritic cells (DCs) are potent APCs. DCs are minor constituents of various immune organs such as spleen, thymus, lymph node, epidermis, and peripheral blood. For instance, DCs represent merely about 1% of crude spleen (*see* Steinman et al. (1979) J. Exp. Med 149: 1) or epidermal cell suspensions (*see* Schuler et al. (1985) J. Exp. Med 161:526; Romani et al. J. Invest. Dermatol (1989) 93: 600) and 0.1-1% of mononuclear cells in peripheral blood (*see* Freudenthal et al. Proc. Natl Acad Sci USA (1990) 87: 7698). Methods for isolating DCs from peripheral blood or bone marrow progenitors are known in the art. (*See* Inaba et al. (1992) J. Exp. Med 175:1157; Inaba et al. (1992) J. Exp, Med 176: 1693-1702; Romani et al. (1994) J. Exp. Med. 180: 83-93; Sallusto et al. (1994) J. Exp. Med 179: 1109-1118)). Preferred methods for isolation and culturing of DCs are described in Bender et al. (1996) J. Immun. Meth. 196:121-135 and Romani et al. (1996) J. Immun. Meth 196:137-151.

Dendritic cells (DCs) represent a complex network of antigen presenting cells that are primarily responsible for initiation of primary immunity and the modulation of immune response. (*See* Avigan, Blood Rev. 13:51-64 (1999); Banchereau et al., Nature 392:245-52 (1998)). Partially mature DCs are located at sites of antigen capture, excel at the internalization and processing of exogenous antigens but are poor stimulators of T cell responses. Presentation of antigen by immature DCs may induce T cell tolerance. (*See* Dhodapkar et al., J Exp Med. 193:233-38 (2001)). Upon activation, DCs undergo maturation characterized by the increased expression of costimulatory molecules and CCR7, the chemokine receptor which promotes migration to sites of T cell traffic in the draining lymph nodes. Tumor or cancer cells inhibit DC development through the secretion of IL-10, TGF-β, and VEGF resulting in the accumulation of immature DCs in the tumor bed that potentially suppress anti-tumor responses. (*See* Allavena et al., Eur. J. Immunol. 28:359-69 (1998); Gabrilovich et al., Clin Cancer Res. 3:483-90 (1997); Gabrilovich et al., Blood 92:4150-66 (1998); Gabrilovich, Nat Rev Immunol 4:941-52 (2004)). Conversely, activated DCs can be generated by cytokine mediated differentiation of DC progenitors *ex vivo.* DC maturation and function can be further enhanced by exposure to the toll like receptor 9 agonist, CPG ODN. Moreover, DCs can be manipulated to present tumor antigens potently stimulate anti-tumor immunity. (*See* Asavaroenhchai et al., Proc Natl Acad Sci USA 99:931-36 (2002); Ashley et al., J Exp Med 186:1177-82 (1997)).

"Foster antigen presenting cells" refers to any modified or naturally occurring cells (wild-type or mutant) with antigen presenting capability that are utilized in lieu of antigen presenting cells ("APC") that normally contact the immune effector cells they are to react with. In other words, they are any functional APCs that T cells would not normally encounter *in vivo.*

It has been shown that DCs provide all the signals required for T cell activation and proliferation. These signals can be categorized into two types. The first type, which gives specificity to the immune response, is mediated through interaction between the T-cell receptor/CD3 ("TCR/CD3") complex and an antigenic peptide presented by a major histocompatibility complex ("MHC") class I or II protein on the surface of APCs. This interaction is necessary, but not sufficient, for T cell activation to occur. In fact, without the second type of signals, the first type of signals can result in T cell anergy. The second type of signals, called costimulatory signals, are neither antigen-specific nor MHC restricted, and can lead to a full proliferation response of T cells and induction of T cell effector functions in the presence of the first type of signals.

Thus, the term "cytokine" refers to any of the numerous factors that exert a variety of effects on cells, for example, inducing growth or proliferation. Non-limiting examples of cytokines include, IL-2, stem cell factor (SCF), IL-3, IL-6, IL-7, IL-12, IL-15, G-CSF, GM-CSF, IL-1 α, IL-1 β, MIP-1 α, LIF, c-kit ligand, TPO, and flt3 ligand. Cytokines are commercially available from several vendors such as, for example, Genzyme Corp. (Framingham, Mass.), Genentech (South San Francisco, CA), Amgen (Thousand Oaks, CA) and Immunex (Seattle, WA). It is intended, although not always explicitly stated, that molecules having similar biological activity as wild-type or purified cytokines *(e.g.,* recombinantly produced cytokines) are intended to be used within the spirit and scope of the invention and therefore are substitutes for wild-type or purified cytokines.

"Costimulatory molecules" are involved in the interaction between receptor-ligand pairs expressed on the surface of antigen presenting cells and T cells. One exemplary receptor-ligand pair is the B7 co-stimulatory molecules on the surface of DCs and its counter-receptor CD28 or CTLA-4 on T cells. (*See* Freeman et al. (1993) Science 262:909-911; Young et al. (1992) J. Clin. Invest 90: 229; Nabavi et al. Nature 360:266)). Other important costimulatory molecules include, for example, CD40, CD54, CD80, and CD86. These are commercially available from vendors identified above.

A "hybrid" cell refers to a cell having both antigen presenting capability and also expresses one or more specific antigens. In one embodiment, these hybrid cells are formed by fusing, *in vitro,* APCs with cells that are known to express the one or more antigens of interest. As used herein, the term "hybrid" cell and "fusion" cell are used interchangeably.

A "control" cell refers to a cell that does not express the same antigens as the population of antigen-expressing cells.

The term "culturing" refers to the *in vitro* propagation of cells or organisms on or in media of various kinds, it is understood that the descendants 30 of a cell grown in culture may not be completely identical (*i.e*., morphologically, genetically, or phenotypically) to the parent cell. By "expanded" is meant any proliferation or division of cells.

An "effective amount" is an amount sufficient to effect beneficial or desired results. An effective amount can be administered in one or more administrations, applications or dosages. For purposes of this invention, an effective amount of hybrid cells is that amount which promotes expansion of the antigenic-specific immune effector cells, *e.g.,* T cells.

An "isolated" population of cells is "substantially free" of cells and materials with which it is associated in nature. By "substantially free" or "substantially pure" is meant at least 50% of the population are the desired cell type, preferably at least 70%, more preferably at least 80%, and even more preferably at least 90%. An "enriched" population of cells is at least 5% fused cells. Preferably, the enriched population contains at least 10%, more preferably at least 20%, and most preferably at least 25% fused cells.

The term "autogeneic", or "autologous", as used herein, indicates the origin of a cell. Thus, a cell being administered to an individual (the "recipient") is autogeneic if the cell was derived from that individual (the "donor") or a genetically identical individual (*i.e.,* an identical twin of the individual). An autogeneic cell can also be a progeny of an autogeneic cell. The term also indicates that cells of different cell types are derived from the same donor or genetically identical donors. Thus, an effector cell and an antigen presenting cell are said to be autogeneic if they were derived from the same donor or from an individual genetically identical to the donor, or if they are progeny of cells derived from the same donor or from an individual genetically identical to the donor.

Similarly, the term "allogeneic", as used herein, indicates the origin of a cell. Thus, a cell being administered to an individual (the "recipient") is allogeneic if the cell was derived from an individual not genetically identical to the recipient. In particular, the term relates to non-identity in expressed MHC molecules. An allogeneic cell can also be a progeny of an allogeneic cell. The term also indicates that cells of different cell types are derived from genetically nonidentical donors, or if they are progeny of cells derived from genetically non-identical donors. For example, an APC is said to be allogeneic to an effector cell if they are derived from genetically non-identical donors.

A "subject" is a vertebrate, preferably a mammal, more preferably a human. Mammals include, but are not limited to, murines, simians, humans, farm animals, sport animals, and pets.

As used herein, "genetic modification" refers to any addition, deletion or disruption to a cell's endogenous nucleotides.

A "viral vector" is defined as a recombinantly produced virus or viral particle that comprises a polynucleotide to be delivered into a host cell, either *in vivo, ex vivo* or *in vitro.* Examples of viral vectors include retroviral vectors, adenovirus vectors, adeno-associated virus vectors and the like. In aspects where gene transfer is mediated by a retroviral vector, a vector construct refers to the polynucleotide comprising the retroviral genome or part thereof, and a therapeutic gene.

As used herein, the terms "retroviral mediated gene transfer" or "retroviral transduction" carries the same meaning and refers to the process by which a gene or a nucleic acid sequence is stably transferred into the host cell by virtue of the virus entering the cell and integrating its genome into the host cell genome. The virus can enter the host cell via its normal mechanism of infection or be modified such that it binds to a different host cell surface receptor or ligand to enter the cell.

Retroviruses carry their genetic information in the form of RNA. However, once the virus infects a cell, the RNA is reverse-transcribed into the DNA form that integrates into the genomic DNA of the infected cell. The integrated DNA form is called a provirus.

In aspects where gene transfer is mediated by a DNA viral vector, such as a adenovirus (Ad) or adeno-associated virus (AAV), a vector construct refers to the polynucleotide comprising the viral genome or part thereof, and a therapeutic gene. Adenoviruses (Ads) are a relatively well characterized, homogenous group of viruses, including over 50 serotypes. (*See, e.g.,* WO 95/27071). Ads are easy to grow and do not integrate into the host cell genome. Recombinant Ad-derived vectors, particularly those that reduce the potential for recombination and generation of wild-type virus, have also been constructed. (*See,* WO 95/00655; WO 95/11984). Wild-type AAV has high infectivity and specificity integrating into the host cells genome. (*See* Hermonat and Muzyczka (1984) PNAS USA 81:6466-6470; Lebkowski et al., (1988) Mol Cell Biol 8:3988-3996).

Vectors that contain both a promoter and a cloning site into which a polynucleotide can be operatively linked are well known in the art. Such vectors are capable of transcribing RNA *in vitro* or *in vivo,* and are commercially available from sources such as Stratagene (La Jolla, CA) and Promega Biotech (Madison, WI). In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression. Examples of suitable vectors are viruses, such as baculovirus and retrovirus, bacteriophage, cosmid, plasmid, fungal vectors and other recombination vehicles typically used in the art which have been described for expression in a variety of eucaryotie and prokaryotic hosts, and may be used for gene therapy as well as for simple protein expression.

Among these are several non-viral vectors, including DNA/liposome complexes, and targeted viral protein DNA complexes. To enhance delivery to a cell, the nucleic acid or proteins of this invention can be conjugated to antibodies or binding fragments thereof which bind cell surface antigens, e.g., TCR, CD3 or CD4. Liposomes that also comprise a targeting antibody or fragment thereof can be used in the methods of this invention. This invention also provides the targeting complexes for use in the methods disclosed herein.

Polynucleotides are inserted into vector genomes using methods well known in the art. For example, insert and vector DNA can be contacted, under suitable conditions, with a restriction enzyme to create complementary ends on each molecule that can pair with each other and be joined together with a ligase. Alternatively, synthetic nucleic acid linkers can be ligated to the termini of restricted polynucleotide. These synthetic linkers contain nucleic acid sequences that correspond to a particular restriction site in the vector DNA. Additionally, an oligonucleotide containing a termination codon and an appropriate restriction site can be ligated for insertion into a vector containing, for example, some or all of the following: a selectable marker gene, such as the neomycin gene for selection of stable or transient transfectants in mammalian cells; enhancer/promoter sequences from the immediate early gene of human CMV for high levels of transcription; transcription termination and RNA processing signals from SV4O for mRNA stability; SV40 polyoma origins of replication and ColEI for proper episomal replication; versatile multiple cloning sites; and T7 and SP6 RNA promoters for *in vitro* transcription of sense and antisense RNA. Other means are well known and available in the art.

As used herein, "expression" refers to the process by which polynucleotides are transcribed into mRNA and translated into peptides, polypeptides, or proteins. If the polynucleotide is derived from genomic DNA, expression may include splicing of the mRNA, if an appropriate eukaryotic host is selected. Regulatory elements required for expression include promoter sequences to bind RNA polymerase and transcription initiation sequences for ribosome binding. For example, a bacterial expression vector includes a promoter such as the lac promoter and for transcription initiation the Shine-Dalgarno sequence and the start codon AUG (Sambrook et al. (1989), *supra*)*.* Similarly, a eukaryotic expression vector includes a heterologous or homologous promoter for RNA polymerase II, a downstream polyadenylation signal, the start codon AUG, and a termination codon for detachment of the ribosome. Such vectors can be obtained commercially or assembled by the sequences described in methods well known in the art, for example, the methods described above for constructing vectors in general.

The terms "major histocompatibility complex" or "MHC" refers to a complex of genes encoding cell-surface molecules that are required for antigen presentation to immune effector cells such as T cells and for rapid graft rejection. In humans, the MHC complex is also known as the HLA complex. The proteins encoded by the MHC complex are known as "MHC molecules" and are classified into class I and class II MHC molecules. Class I MHC molecules include membrane heterodimeric proteins made up of an α chain encoded in the MHC associated noncovalently with β2-microglobulin. Class I MHC molecules are expressed by nearly all nucleated cells and have been shown to function in antigen presentation to CD8+ T cells. Class I molecules include HLA-A, -B, and -C in humans. Class II MHC molecules also include membrane heterodimeric proteins consisting of noncovalently associated and J3 chains. Class II MHCs are known to function in CD4+ T cells and, in humans, include HLA-DP, -DQ, and DR. The term "MHC restriction" refers to a characteristic of T cells that permits them to recognize antigen only after it is processed and the resulting antigenic peptides are displayed in association with either a class I or class II MHC molecule. Methods of identifying and comparing MHC are well known in the art and are described in Allen M. et al. (1994) Human Imm. 40:25-32; Santamaria P. et al. (1993) Human Imm. 37:39-50; and Hurley C.K. et al. (1997) Tissue Antigens 50:401-415.

The term "sequence motif' refers to a pattern present in a group of 15 molecules (*e*.*g*., amino acids or nucleotides). For instance, in one embodiment, the present invention provides for identification of a sequence motif among peptides present in an antigen. In this embodiment, a typical pattern may be identified by characteristic amino acid residues, such as hydrophobic, hydrophilic, basic, acidic, and the like.

The term "peptide" is used in its broadest sense to refer to a compound of two or more subunit amino acids, amino acid analogs, or peptidomimetics. The subunits may be linked by peptide bonds. In another embodiment, the subunit may be linked by other bonds, e.g. ester, ether, etc.

As used herein the term "amino acid" refers to either natural and/or 25 unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is commonly called a polypeptide or a protein.

As used herein, "solid phase support" is used as an example of a "carrier" and is not limited to a specific type of support. Rather a large number of supports are available and are known to one of ordinary skill in the art. Solid phase supports include silica gels, resins, derivatized plastic films, glass beads, cotton, plastic beads, alumina gels. A suitable solid phase support may be selected on the basis of desired end use and suitability for various synthetic protocols. For example, for peptide synthesis, solid phase support may refer to resins such as polystyrene (*e.g.,* PAM-resin obtained from Bachem Inc., Peninsula Laboratories, etc.), PoLYHIPE® resin (obtained from Aminotech, Canada), polyamide resin (obtained from Peninsula Laboratories), polystyrene resin grafted with polyethylene glycol (TentaGel®, Rapp Polymere, Tubingen, Germany) or polydimethylacrylamide resin (obtained from MilligenlBiosearch, California). In a preferred embodiment for peptide synthesis, solid phase support refers to polydimethylacrylamide resin.

The term "aberrantly expressed" refers to polynucleotide sequences in a cell or tissue which are differentially expressed (either over-expressed or under-expressed) when compared to a different cell or tissue whether or not of the same tissue type, *i.e*., lung tissue versus lung cancer tissue.

"Host cell" or "recipient cell" is intended to include any individual cell or cell culture which can be or have been recipients for vectors or the incorporation of exogenous nucleic acid molecules, polynucleotides and/or proteins. It also is intended to include progeny of a single cell, and the progeny may not necessarily be completely identical (in morphology or in genomic or total DNA complement) to the original parent cell due to natural, accidental, or deliberate mutation. The cells may be prokaryotic or eukaryotic, and include but are not limited to bacterial cells, yeast cells, animal cells, and mammalian cells, e.g., murine, rat, simian or human.

An "antibody" is an immunoglobulin molecule capable of binding an antigen. As used herein, the term encompasses not only intact immunoglobulin molecules, but also anti-idiotypic antibodies, mutants, fragments, fusion proteins, humanized proteins and modifications of the immunoglobulin molecule that comprise an antigen recognition site of the required specificity.

An "antibody complex" is the combination of antibody and its binding partner or ligand.

A "native antigen" is a polypeptide, protein or a fragment containing an epitope, which induces an immune response in the subject.

The term "isolated" means separated from constituents, cellular and otherwise, in which the polynucleotide, peptide, polypeptide, protein, antibody, or fragments thereof, are normally associated with in nature. As is apparent to those of skill in the art, a non-naturally occurring polynucleotide, peptide, polypeptide, protein, antibody, or fragments thereof, does not require "isolation" to distinguish it from its naturally occurring counterpart. In addition, a "concentrated", "separated" or "diluted" polynucleotide, peptide, polypeptide, protein, antibody, or fragments thereof, is distinguishable from its naturally occurring counterpart in that the concentration or number of molecules per volume is greater than "concentrated" or less than "separated" than that of its naturally occurring counterpart. A polynucleotide, peptide, polypeptide, protein, antibody, or fragments thereof, which differs from the naturally occurring counterpart in its primary sequence or for example, by its glycosylation pattern, need not be present in its isolated form since it is distinguishable from its naturally occurring counterpart by its primary sequence, or alternatively, by another characteristic such as glycosylation pattern. Although not explicitly stated for each of the inventions disclosed herein, it is to be understood that all of the above embodiments for each of the compositions disclosed below and under the appropriate conditions, are provided by this invention. Thus, a non-naturally occurring polynucleotide is provided as a separate embodiment from the isolated naturally occurring polynucleotide. A protein produced in a bacterial cell is provided as a separate embodiment from the naturally occurring protein isolated from a eucaryotic cell in which it is produced in nature.

A "composition" is intended to mean a combination of active agent and another compound or composition, inert (for example, a detectable agent, carrier, solid support or label) or active, such as an adjuvant.

A "pharmaceutical composition" is intended to include the combination of an active agent with a carrier, inert or active, making the composition suitable for diagnostic or therapeutic use *in vitro, in vivo* or *ex vivo.*

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin, REMINGTON'S PHARM. SCI, 15th Ed. (Mack Publ. Co., Easton (1975)).

As used herein, the term "inducing an immune response in a subject" is a term well understood in the art and intends that an increase of at least about 2-fold, more preferably at least about 5-fold, more preferably at least about 10-fold, more preferably at least about 100-fold, even more preferably at least about 500-fold, even more preferably at least about 1000-fold or more in an immune response to an antigen (or epitope) can be detected (measured), after introducing the antigen (or epitope) into the subject, relative to the immune response (if any) before introduction of the antigen (or epitope) into the subject. An immune response to an antigen (or epitope), includes, but is not limited to, production of an antigen-specific (or epitope-specific) antibody, and production of an immune cell expressing on its surface a molecule which specifically binds to an antigen (or epitope). Methods of determining whether an immune response to a given antigen (or epitope) has been induced are well known in the art. For example, antigen specific antibody can be detected using any of a variety of immunoassays known in the art, including, but not limited to, ELISA, wherein, for example, binding of an antibody in a sample to an immobilized antigen (or epitope) is detected with a detectably-labeled second antibody (*e.g*., enzyme-labeled mouse anti-human Ig antibody). Immune effector cells specific for the antigen can be detected any of a variety of assays known to those skilled in the art, including, but not limited to, FACS, or, in the case of CTLs, ⁵¹CR-release assays, or ³H-thymidine uptake assays.

### Fusions

DCs can be obtained from bone marrow cultures, peripheral blood, spleen, or any other appropriate tissue of a mammal using protocols known in the art. Bone marrow contains DC progenitors, which, upon treatment with cytokines, such as granulocyte-macrophage colony-stimulating factor ("GM-CSF") and interleukin 4 ("IL-4"), proliferate and differentiate into DCs. Tumor necrosis cell factor (TNF) is optionally used alone or in conjunction with GM-CSF and/or IL-4 to promote maturation of DCs. DCs obtained from bone marrow are relatively immature (as compared to, for instance, spleen DCs). GM-CSF/IL-4 stimulated DC express MHC class I and clasps II molecules, B7-1, B7-2, ICAM, CD40 and variable levels of CD83. These immature DCs are more amenable to fusion (or antigen uptake) than the more mature DCs found in spleen, whereas more mature DCs are relatively more effective antigen presenting cells. Peripheral blood also contains relatively immature DCs or DC progenitors, which can propagate and differentiate in the presence of appropriate cytokines such as GM-CSF and-which can also be used in fusion.

The non-dendritic cells used in the invention can be derived from any tissue or cancer (including, but not limited to, breast cancer, lung, pancreatic cancer, prostate cancer, renal cancer, bladder cancer, neurological cancers, genitourinary cancers, hematological cancers, melanoma and other skin cancers, gastrointestinal cancers, and brain tumors (*i.e.,* gliomas) by well known methods and can be immortalized. Non-dendritic cells expressing a cell-surface antigen of interest can be generated by transfecting the non-dendritic cells of a desired type with a nucleic acid molecule that encodes a polypeptide comprising the antigen. Exemplary cell-surface antigens are MUC1, α-fetoprotein, γ-fetoprotein, carcinoembryonic antigen, fetal sulfoglycoprotein antigen, α₂H-ferroprotein, placental alkaline phosphatase, and leukemia-associated membrane antigen. Methods for transfection and identifying antigens are well known in the art.

If the non-dendritic cells die or at least fail to proliferate in the presence of a given reagent and this sensitivity can be overcome by the fusion with DCs, the post-fusion cell mixtures containing the fused as well as the parental cells may optionally be incubated in a medium containing this reagent for a period of time sufficient to eliminate most of the unfused cells. For instance, a number of tumor cell lines are sensitive to HAT due to lack of functional hypoxanthine-guanine phosphoribosyl transferase ("HGPRT"). Fused cells formed by DCs and these tumor cell lines become resistant to HAT, as the DCs contribute functional HGPRT. Thus, a HAT selection can be performed after fusion to eliminate unfused parental cells. Contrary to standard HAT selection techniques, the HAT selection generally should not last for more than 12 days, since lengthy culturing leads to loss of MHC class II protein and/or B7 costimulatory molecules on the fused cells. The fusion product is used directly after the fusion process (*e.g*., in antigen discovery screening methods or in therapeutic methods) or after a short culture period.

Fused cells are optionally irradiated prior to clinical use. Irradiation induces expression of cytokines, which promote immune effector cell activity.

In the event that the fused cells lose certain DC characteristics such as expression of the APC-specific T-cell stimulating molecules, primary fused cells can be refused with dendritic cells to restore the DC phenotype. The refused cells (*i.e*., secondary fused cells) are found to be highly potent APCs. The fused cells can be refused with the dendritic or non-dendritic parental cells as many times as desired.

Fused cells that express MHC class II molecules, B7, or other desired T-cell stimulating molecules can also be selected by panning or fluorescence-activated cell sorting with antibodies against these molecules.

Cells infected with an intracellular pathogen can also be used as the non-dendritic partner of the fusion for treatment of the disease caused by that pathogen. Examples of pathogens include, but are not limited to, viruses (*e.g*., human immunodeficiency virus; hepatitis A, B, or C virus; papilloma virus; herpes virus; or measles virus), bacteria (*e.g*., *Corynebacterium diphtheria, Bordetella pertussis*), and intracellular eukaryotic parasites (*e.g., Plasmodiuin spp., Schistosoina spp., Leishmania spp., Trypanosoma spp.,* or *Mycobacterium lepre*).

Alternatively, non-dendritic cells transfected with one or more nucleic acid constructs each of which encodes one or more identified cancer antigens or antigens from a pathogen can be used as the non-dendritic partner in fusion. These antigens need not be expressed on the surface of the cancer cells or pathogens, so long as the antigens can be presented by a MHC class I or II molecule on the fused cells.

### Methods of Making the Fusions

Fusion between the DCs and the non-dendritic cells can be carried out with well-known methods such as those using polyethylene glycol ("PEG"), Sendai virus, or electrofusion. DCs are autologous or allogeneic. (*See, e*.*g*., U.S. Patent No. 6,653,848, which is herein incorporated by reference in its entirety). The ratio of DCs to non-dendritic cells in fusion can vary from 1:100 to 1000:1, with a ratio higher than 1:1 being preferred where the nondendritic cells proliferate heavily in culture. Most preferably, the ratio is 1:1, 5:1, or 10:1. After fusion, unfused DCs usually die off in a few days in culture, and the fused cells can be separated from the unfused parental non-dendritic cells by the following two methods, both of which yield fused cells of approximately 50% or higher purity, *i.e.,* the fused cell preparations contain less than 50%, and often less than 30%, unfused cells.

Specifically, one method of separating unfused cells from fused cells is based on the different adherence properties between the fused cells and the non-dendritic parental cells. It has been found that the fused cells are generally lightly adherent to tissue culture containers. Thus, if the non-dendritic parental cells are much more adherent, e.g., in the case of carcinoma cells, the post-fusion cell mixtures can be cultured in an appropriate medium (HAT is not needed but may be added if it slows the growth of unfused cells) for a short period of time (*e.g.,* 5-10 days). Subsequently, the fused cells can be gently dislodged and aspirated off, while the unfused cells grow firmly attached to the tissue culture containers. Conversely, if the non-dendritic parental cells grow in suspension, after the culture period, they can be gently aspirated off while leaving the fused cells loosely attached to the containers. Alternatively, the hybrids are used directly without an *in vitro* cell culturing step. It has been shown that fused cells lack functional hypoxanthine-guanine phosphoribosyl transferase ("HGPRT") enzyme and are, therefore, resistant to treatment with the compound HAT. Accordingly, to select these cells HAT can be added to the culture media. However, unlike conventional HAT selection, hybrid cell cultures should not be exposed to the compound for more than 12 days.

Fused cells obtained by the above-described methods typically retain the phenotypic characteristics of DCs. For instance, these fused cells express T-cell stimulating molecules such as MHC class II protein, B7-1, B7-2, and adhesion molecules characteristic of APCs such as ICAM-1. The fused cells also continue to express cell-surface antigens of the parental non-dendritic cells, and are therefore useful for inducing immunity against the cell-surface antigens. Notably, when the non-dendritic fusion partner is a tumor cell, the tumorigenicity of the fused cell is often found to be attenuated in comparison to the parental tumor cell.

In the event that the fused cells lose certain DC characteristics such as expression of the APC-specific T-cell stimulating molecules, they (*i.e.,* primary fused cells) can be re-fused with dendritic cells to restore the DC phenotype. The re-fused cells (*i.e.,* secondary fused cells) are found to be highly potent APCs, and in some cases, have even less tumorigenicity than primary fused cells. The fused cells can be re-fused with the dendritic or non-dendritic parental cells as many times as desired.

Alternatively, non-dendritic cells transfected with one or more nucleic acid constructs, each of which encodes one or more identified cancer antigens or antigens from a pathogen, can be used as the non-dendritic partner in fusion. These antigens need not be expressed on the surface of the cancer cells or pathogens, so long as the antigens can be presented by a MHC class I or II molecule on the fused cells.

### Methods of Using the Fusions

The fused cells of the invention can be used to stimulate the immune system of a mammal for treatment or prophylaxis of a disease. For instance, to treat a primary or metastatic tumor in a human, a composition containing fused cells formed by his own DCs and tumor cells can be administered to him, e.g., at a site near the lymphoid tissue. The composition may be given multiple times (*e.g*., three to five times) at an appropriate interval (*e.g*., every two to three weeks) and dosage (*e.g*., approximately 10⁵-10⁸, *e*.*g*.,about 0.5 X 10⁶ to 1 X 14⁶, fused cells per administration). For prophylaxis (*i.e.,* vaccination) against cancer, non-syngeneic fused cells such as those formed by syngeneic DCs and allogeneic or xenogeneic cancer cells, or by allogeneic DCs and cancer cells, can be administered. To monitor the effect of vaccination, cytotoxic T lymphocytes obtained from the treated individual can be tested for their potency against cancer cells in cytotoxic assays. Multiple boosts may be needed to enhance the potency of the cytotoxic T lymphocytes.

Compositions containing the appropriate fused cells are administered to an individual (*e.g.,* a human) in a regimen determined as appropriate by a person skilled in the art. For example, the composition may be given multiple times (*e.g*., three to five times) at an appropriate interval (*e.g.,* every two to three weeks) and dosage (*e.g.,* approximately 10⁵-10⁸, preferably about 10⁷ fused cells per administration).

Fused cells generated by DCs and these transfected cells can be used for both treatment and prophylaxis of cancer or a disease caused by that pathogen. By way of non-limiting example, fusion cells expressing MUC1 can be used to treat or prevent breast cancer, ovarian cancer, pancreatic cancer, prostate gland cancer, lung cancer, lymphoma, certain leukemias, and myeloma; fusion cells expressing α-fetoprotein can be used to treat or prevent hepatoma or chronic hepatitis, where α-fetoprotein is often expressed at elevated levels; and fusion cells expressing prostate-specific antigen can be used to treat prostate cancer. Administration of compositions containing the fused cells so produced is as described above.

Tumor cells suppress host immunity, in part, by disrupting the development and function of antigen presenting cells. Thus, a potential issue concerning the effectiveness of the DC/tumor fusion vaccine is that the tumor cell fusion partner will inhibit DC differentiation and interfere with antigen presentation by the fusion vaccine.

DC/tumor fusions express a broad array of tumor antigens presented in the context of DC mediated costimulation and are highly effective in generating anti-tumor immunity. Endogenously and internalized antigens are presented in the context of the MHC class I and II pathways resulting in a balanced helper and cytotoxic T lymphocyte response. (*See* Parkhurst et al., J Immunol 170:5317-25 (2003)). In animal models, vaccination with DC/tumor fusions results protects against an otherwise lethal challenge of tumor cells and effectively eradicates established disease. (*See* Gong et al., Nat Med 3:558-61 (1997); Gong et al, Proc Natl Acad Sci USA 95:6279-83 (1998); Gong et al., Blood 99:2512-17 (2002); Lespagnard et al., Int J Cancer 76:250-58 (1998)). In fact, fusions of patient-derived breast carcinoma cells and DC stimulated T cell mediated lysis of autologous tumor cells *in vitro.* (*See* Gong et al., Proc Natl Acad Sci USA 97:2715-18) (2000)).

However, in a clinical trial for patients with metastatic breast carcinoma, vaccination with autologous DC/tumor fusions induced anti-tumor irnrnunity in a majority of patients, while clinical responses were observed in a only subset of patients. (*See* Avigan et al., Clin Cancer Res 10:4699-708 (2004); Avigan et al., J Clin Oncol ASCO Annual Meeting Proceedings 22:169 (2004)). In this phase I/II trial, 23 patients with metastatic breast and renal carcinoma underwent vaccination with partially mature DCs fused with autologous tumor cells harvested from sites of accessible tissue. (*See* Avigan et al., Clin Cancer Res. 10:4699-708 (2004)). Fusion cells demonstrated coexpression of tumor specific antigens such as MUC-1 and DC-derived costimulatory molecules, while vaccination resulted in anti-tumor immune responses in 10/18 evaluable patients as manifested by an increase in IFNγ following *ex vivo* exposure to tumor lysate, two patients demonstrated disease regression and six patients had stabilization of metastatic disease. Therefore, although the vaccination with DC/breast cancer fusions stimulated anti-tumor immune responses in a majority of patients, only a subset demonstrated a clinically meaningful disease response.

The phenotypic characteristics of DC/breast carcinoma fusions have been examined with respect to their function as antigen presenting cells. (*See* Vasir et al., Br. J. Hematol. 129:687-700 (2005)). Specifically, fusion of DCs with breast carcinoma cells resulted in enhanced expression of the costimulatory markers, CD80, CD86, and the maturation marker CD83. Fusion cells generated with immature and mature DCs demonstrated similar levels of maturation, thereby suggesting that the fusion process itself promoted DC activation. Indeed, significant expression of IL-12 was observed in both populations consistent with their role as potent antigen presenting cells with the capacity to stimulate primary immune responses. Expression of CCR7 by the fusion cell populations supports their capacity to stimulate to sites of T cell traffic in the draining lymph node. DC/breast carcinoma fusions also potently stimulated autologous T cell proliferation with an associated secretion of high levels of IFNγ.

Thus, immature DCs undergo maturation following PEG mediated fusion with breast carcinoma cells and demonstrate similar functional characteristics to mature DC/breast carcinoma fusions. However, these DC/tumor fusions stimulate a mixed response of activated and regulatory T cells. Stimulation with fusion cells resulted in an increase of CD4/CD25+ cells. Immunophenotyping of this population revealed the presence of activated (CD69+) as well as inhibitory (CTLA-4+, Foxp3) T cells. Moreover, a relative increase in both IFNγ and IL-10 producing cells was also observed.

Tumor cells create an immunosuppressive environment characterized by ineffective T cell function as well as the increased presence of regulatory T cells that dampen immune activation and potentially limit the response to cancer vaccines. (*See* Baecher-Allan et al., J Immunol 167:1245-53 92001); Dieckmann et al., J Exp Med. 193:1303-10 (2001); Jonuleit et al, J Exp Med. 193:128594 (2001)). The increased presence of regulatory T cells has been noted in the circulation, draining lymph nodes, and tumor beds of cancer patients at levels that correlate with disease burden. *(See* Liyanage et al., J Immunol. 169:2756-61 (2002); Sasada et al., Cancer 98:1089-99 (2003); Ormandy et al., Cancer Res. 65:2457-64 (2005)).

Cancer vaccine therapy relies on the ability of a vaccine to stimulate tumor-specific T cell responses *in vivo.* Often, effector cell dysfunction in patients with malignancy limits cancer vaccine efficacy and efficiency. Thus, a major challenge in developing an effective cancer vaccine strategy is overcoming the intrinsic immune deficiencies that limit immunologic response in tumor bearing patients. To be effective, a cancer vaccine must demonstrate the capacity to present tumor antigens in the context of stimulatory signaling, migrate to sites of T cell traffic, and induce the expansion of activated effector cells with the ability to lyse tumor targets.

Two central elements of tumor mediated immune suppression include inhibition of DC maturation and the increased presence of regulatory T cells. (*See* Gabrilovich et al, Clin Cancer Res 3:483-90 (1997); Gabrilovich et at., Blood 92:4150-66 (1998); Gabrilovich, Nat Rev Immunol 4:941-52 (2004)).

One concern regarding the DC/tumor or cancer cell fusions is that tumor cells in the vaccine preparation may inhibit its function as an antigen presenting cell. Another potential issue limiting response to vaccination is the increased presence of regulatory T cells that suppress T cell activation.

Regulatory T cells play a significant role in mediated tolerance to self antigens in the normal host. In patients with malignancy, their increased presence is thought to mediate tumor associated suppression of host immune responses. (*See* Baecher-Allan et al., J Immunol. 167:1245-53 (2001); Piccirillo et al., J Immunol 167:1137-40 (2001); Wood et al., Nat Rev Immunol. 3:199-210 (2003)). Precise definition of regulatory T cells is complex, as many markers such as GITR and CD25 are shared between regulatory and activated T cell populations. Regulatory cells are identified by a panel of markers including CD25^{high}, GITR, CTLA-4, and Foxp3; a lack of response to mixed lymphocyte reactions; and the ability to suppress autologous T cell responses *in vitro.*

Regulatory T cells deliver inhibitory signals via direct cell contact and the release of cytokines that play a role in mediating tumor associated anergy. As noted, regulatory T cells are increased in the circulation, tumor bed, and lymph nodes of patients with malignancy, and their presence has been associated with worse outcomes. (*See* Curiel et al., Nat Med 10:942-49 (2004)); Liyanage et al., J. Immunol 169:2756-61 (2002); Ormandy et al., Cancer Res 65:2457-64 (2005)).

Paradoxically, studies have demonstrated that vaccination with DC/cancer cell fusions may lead to the expansion of regulatory T cells that ultimately blunt the immune response. (*See* Javia et al., J Imunother 26:85-93 (2003)). For example, in animal models, the depletion of regulatory T cells or the activation of innate immunity through ligation of the toll like receptors (TLR) resulted in enhanced response to tumor vaccines. (*See* Prasa et al., J Immunol 174:90-98 (2003); Casares et al., J Immunol 171:5931-39 (2003); Tanaka et al., J Immunother. 25:207-17 (2002); Dannull et al., J. Clin Invest 15:3623-33 (2005)). Moreover, ligation of the T cell/costimulatory complex (CD3/CD28) has also been shown to promote the activation of T cells when administered in the context of other stimulatory signals. (*See* Jung et al., Blood 102:3439-45 (2003)). Thus, the presence of regulatory T cells may prevent response to active immunization in patients with malignancy.

In previous studies, vaccination with antigen pulsed immature DCs induced tolerance in antigen specific T cells. (*See* Dhodapkar et al., J Exp Med. 193:233-38 (2001)). Moreover, fusion of immature DCs with multiple myeloma cells resulted in the further maturation of the DC fusion partner. (*See* Vasir et al., Br J Ahematol. 129:687-700 (2005)).

These results provide a strong rational for examining the *ex vivo* use of vaccines to generate functionally active T cells. In adoptive T cell transfer, the number of regulatory T cells can be modified, and an antigen specific population of effector cells can be transferred. Studies in patients with metastatic melanoma have shown that the transfer of autologous melanoma reactive tumor infiltrating lymphocytes (TILs) following lymphodepletion results in sustained clinical responses. (*See* Zhou et al., J Immunother. 28:53-62 (2005)). These studies have also shown that adoptive transfer of tumor-reactive T cells following removal of tumor suppressor cells induces tumor regression in 50% of patients with advanced disease. (*See* Robbins et al., J Immunol. 173:7125-30 (2004)). However, this use of TILs is limited to a small number of tumors types where they are obtainable. Therefore, utilizing T cells that have been expanded *ex vivo* by tumor vaccines for adoptive immunotherapy remains a focus of great interest.

### Educated T Cells

This invention also provides populations of educated, antigen-specific immune effector cells expanded in culture at the expense of hybrid cells, wherein the hybrid cells comprise antigen presenting cells (APCs) fused to cells that express one or more antigens. In one embodiment, the APC are dendritic cells (DCs) and the hybrid cells are expanded in culture. In another embodiment, the cells expressing the antigen(s) are tumor cells and the immune effector cells are cytotoxic T lymphocytes (CTLs). The DCs can be isolated from sources such as blood, skin, spleen, bone marrow or tumor. Methods for preparing the cell populations also are provided by this invention.

Any or all of the antigen-specific immune effector cells or the hybrid cells of the invention can be or have been genetically modified by the insertion of an exogenous polynucleotide. As an example, the polynucleotide introduced into the cell encodes a peptide, a ribozyme, or an antisense sequence.

The cells expressing the antigen(s) and the immune effector cells may have been enriched from a tumor. In a further embodiment, the immune effector cells are cytotoxic T lymphocytes (CTLs). The method also provides the embodiment wherein the APCs and the antigen-expressing cells are derived from the same subject or from different subjects (*i.e.,* autologous or allogeneic).

In a further modification of this method, the immune effector cells are cultured in the presence of a cytokine, e.g., IL-2 or GM-CSF and/or a costimulatory molecule.

The hybrid cells used in the present invention may be formed by any suitable method known in the art. In one embodiment, a tumor biopsy sample is minced and a cell suspension created. Preferably, the cell suspension is separated into at least two fractions -- one enriched for immune effector cells, *e.g.,* T cells, and one enriched for tumor cells. Immune effector cells also can be isolated from bone marrow, blood or skin using methods well known in the art.

In general, it is desirable to isolate the initial inoculation population from neoplastic cells prior to culture. Separation of the various cell types from neoplastic cells can be performed by any number of methods, including, for example, the use of cell sorters, magnetic beads, and packed columns. Other procedures for separation can include, but are not limited to, physical separation, magnetic separation, using antibody-coated magnetic beads, affinity chromatography, cytotoxic agents joined to a monoclonal antibody or used in conjunction with a monoclonal antibody, including, but not limited to, complement and cytotoxins, and "panning" with antibody attached to a solid matrix, e.g., plate, elutriation or any other convenient technique known to those skilled in the art.

The use of physical separation techniques include, but are not limited to, those based on differences in physical (density gradient centrifugation and counter-flow centrifugal elutriation), cell surface (lectin and antibody affinity), and vital staining properties (mitochondria-binding dye rho 123 and DNA-binding dye Hoechst 33342). Suitable procedures are well known to those of skill in this art.

Monoclonal antibodies are another useful reagent for identifying markers associated with particular cell lineages and/or stages of differentiation can be used. The antibodies can be attached to a solid support to allow for crude separation. The separation techniques employed should maximize the retention of viability of the fraction to be collected. Various techniques of different efficacy can be employed to obtain "relatively crude" separations. Such separations are up to 10%, usually not more than about 5%, preferably not more than about 1%, of the total cells present not having the marker can remain with the cell population to be retained. The particular technique employed will depend upon efficiency of separation, associated cytotoxicity, ease and speed of performance, and necessity for sophisticated equipment and/or technical skill.

Another method of separating cellular fractions is to employ culture conditions, which allow for the preferential proliferation of the desired cell populations. For example, the fraction enriched for antigen expressing cells is then fused to APCs, preferably dendritic cells. Fusion between the APCs and antigen-expressing cells can be carried out with any suitable method, for example using polyethylene glycol (PEG), electrofusion, or Sendai virus. The hybrid cells are created using the PEG procedure described by Gong et al. (1997) Nat. Med 3(5):558-561, or other methods known in the art..

Precommitted DCs are isolated, for example using metrizamide gradients; nonadherence/adherence techniques (*see* Freduenthal, PS et al. (1990) PNAS 87:7698-7702); percoll gradient separations (*see* Mehta-Damani et al (1994) J. Immunol 153:996-1003) and fluorescence-activated cell sorting techniques (*see* Thomas et al. (1993) J. Immunol 151:6840-6852). In one embodiment, the DCs are isolated essentially as described in WO 96/23060 using FACS techniques. Although there is no specific cell surface marker for human DCs, a cocktail of 20 markers *(e.g.* HLA-DR, B7.2, CD 13/33, *etc*.) are known to be present on DCs. In addition, DCs are known to lack CD3, CD2O, CD56 and CD14 antigens. Therefore, combining negative and positive FACS techniques provides a method of isolating DCs.

The APCs and cells expressing one or more antigens may be autologous, *i*.*e*., derived from the same subject from which that tumor biopsy was obtained. The APCs and cells expressing the antigen may also be allogeneic, *i.e.,* derived from a different subject, since dendritic cells are known to promote the generation of primary immune responses.

### Expansion of Antigen-Specific Immune Effector Cells

The present invention makes use of these hybrid cells to stimulate production of an enriched population of antigen-specific (*i.e.,* "educated") immune effector cells. The antigen-specific immune effector cells are expanded at the expense of the hybrid cells, which die in the culture. The process by which naive immune effector cells become educated by other cells is described essentially in Coulie, Molec. Med Today 261-268 (1997).

Hybrid cells prepared as described above are mixed with naive immune effector cells. Preferably, the immune effector cells specifically recognize tumor cells and have been enriched from the tumor biopsy sample as described above. Optionally, the cells may be cultured in the presence of a cytotokine, for example IL-2. Because DCs secrete potent immunostimulatory cytokines, such as IL-12, it may not be necessary to add supplemental cytokines during the first and successive rounds of expansion. However, if fused cells are not making IL-12, this cytokine is added to the culture. In any event, the culture conditions are such that the antigen-specific immune effector cells expand (*i.e.,* proliferate) at a much higher rate than the hybrid cells Multiple infusions of hybrid cells and optional cytokines can be performed to further expand the population of antigen-specific cells.

The addition of a second stimulatory signal decreases the fusion mediated expansion of regulatory T cells, and, thus, favors the development of an activated anti-tumor immune response. Suitable secondary stimulatory signals include, but are not limited to, IL-12; IL-18; the TLR 9 agonist, CPG-ODN; and anti-CD3/CD28.

For example, animals models have demonstrated that co-administration of IL-12 promotes the efficacy of the DC/tumor fusion vaccine. (*See* Akasaki et al., J Immunother. 24:106-113 (2001)). Another strategy to minimize the effect of regulatory T cells is through the activation of innate immunity by ligation of the toll like receptors (TLRs). In an animal model, administration of CPG ODN to activate TLR9 was shown to overcome the immunosuppression resulting from an expanding tumor burden. Exposure to CPG decreased the presence of regulatory cells and promoted vaccine response. Moreover, exposure to the TLR 7/8 agonist resulted in enhanced DC activation as manifested by increased expression of costimulatory and maturation markers. Likewise, addition of the TLR 9 agonists (CpG), IL-12 and IL-18 reduced the levels of regulatory T cells following fusion mediated stimulation.

It has previously been demonstrated that DC/tumor fusions stimulate tumor reactive T cells with the capacity to lyse autologous tumor targets. Moreover, previous studies have also demonstrated that primary exposure to anti-CD3/CD28 restores the complexity of the T cell repertoire potentially enhancing the capacity of the DC/tumor fusions to expand tumor reactive clones. In contrast, secondary exposure to anti-CD3/CD28 following fusion mediated stimulation may result in the more specific expansion of activated, tumor reactive cells.

Ligation of CD3/CD28 provides a powerful antigen independent stimulus mediated by the T cell receptor/costimulatory complex resulting in the activation of signaling pathways including NFκB. *(See* Bonyhadi et al., J. Immunol. 174:2366-75 (2005); Wang et al., Mol Cell Biol. 24:164-71 (2004); Herndon et al., J Immunol. 166:5654-64 (2001); Khoshnan et al., J Immunol 165:6933-40 (2000); and Yamada-Ohnishi et al., Stem Cells Dev 13:315-22 (2004)). This process delivers a strong activation and proliferation signal which induces T cell expansion and enhanced complexity of the T cell repertoire in patients with HIV and malignancy. (*See* Bonyhadi et al., J. Immunol. 174:2366-75 (2005); Kalamasz et al., J Immunother. 27:405-18 (2004)). T cells expanded *ex vivo* with anti-CD3/CD28 have been explored as a potential strategy to reverse tumor associated cellular immune dysfunction. However, exposure to anti-CD3/CD28 alone may expand activated or suppressor cells dependent on the associated cytokine milieu. (*See* Jung et al., Blood 102:3439-46 (2003)).

The effect of anti-CD3/CD28 stimulation on T cell phenotype is complex and results in diverse and contradictory effects dependent on the model being examined. Exposure to anti-CD3/CD28 promotes the expansion of activated or suppressor T cells dependent on the nature of the immunologic milieu. (*See* Jung et al., Blood 102:3439-46 (2003)). For example, stimulation with anti-CD3/CD28 and IL-15 results in the expansion of regulatory T cells that demonstrate an inhibitory phenotype. (*See* Lin et al., Bone Marrow Transplant 37:881-87 (2006)). In a graft versus host disease model, polarization towards a Th1 or Th2 phenotype following anti-CD3/CD28 stimulation is determined by cytokine exposure (*See* Jung et al., Blood 102:3439-46 (2003)). CD4+ cells cocultured with anti-CD3/CD28, IL-4, and IL-2 secrete increased levels of IL-4 and IL-10. In contrast, in an animal model, exposure of antigen specific T cells to anti-CD3/CD28 resulted in the expansion of memory effector cells that expressed IFNγ upon exposure to antigen and were protective against tumor challenge. (*See* Hughes et al., Cytotherapy 7:396-407 (2005)).

Thus, it has been hypothesized that DC/tumor fusions would provide a unique platform for anti-CD3/CD28 mediated expansion by selectively stimulating activated T cells directed against tumor associated antigens. As such, sequential stimulation with fusions and anti-CD3/CD28 potentially allows for the generation of significant yields of tumor-reactive T cells while minimizing the presence of regulatory T cells in the expanded population. The phenotypic and functional characteristics of T cells that have undergone *in vitro* stimulation with DCs fused with renal carcinoma cells (RCC) or patient derived myeloid leukemia cells has been studied. Moreover, sequential stimulation with DC/breast carcinoma followed by anti-CD3/CD28 resulted in a T cell population that primarily manifested an activated phenotype that was consistent with that of memory effector cells.

Thus, DC/tumor fusions and anti-CD3/CD28 provide a synergistic effect in dramatically expanding anti-tumor T cells with an activated phenotype. It has also been demonstrated in both RCC and breast cancer models that sequential stimulation with DC/tumor fusions and anti-CD3/CD28 resulted in the dramatic expansion of memory effector T cells that was far in excess to that observed following stimulation with DC/RCC fusions or anti-CD3/CD28 alone.

Moreover, fusion stimulated T cells that underwent subsequent anti-CD3/CD28 expansion demonstrated a marked increase in MUC1 reactive T cell clones suggesting that tumor reactive clones that were primed during culture with the fusion cells were subsequently being expanded. Sequential stimulation with DC/tumor fusions followed by anti-CD3/CD28 results in the relatively selective expansion of activated T cells as manifested by significantly increased yields of CD4+/CD25+ cells that expressed CD69 and IFNγ. A more modest increase in cells expressing IL-10 and Foxp3 suggested that expansion of inhibitory populations occurred. As a measure of cytolytic capacity, T cells stimulated by DC/tumor fusions followed by anti-CD3/CD28 demonstrated high levels of granzyme B expression, in excess of that observed following stimulation with fusion cells or anti-CD3/CD28 alone.

Because sequential stimulation with DC/tumor fusions followed by anti-CD3/CD28 results in the dramatic expansion of tumor reactive lymphocytes with a predominant activated phenotype, this strategy provides an ideal platform for adoptive immunotherapy. Moreover, those skilled in the art will recognize that additional approaches to further deplete regulatory T cells in the expanded population might further enhance cancer vaccine efficacy.

Using the hybrid cells as described, a potent antigen-specific population of immune effector cells can be obtained. These cells can be T cells that are specific for tumor-specific antigens.

### Methods of Using Educated T Cells

As described herein, an effective amount of the cells described herein can be administered to a subject to provide adoptive immunotherapy. An effective amount of cytokine or other costimulatory molecule also can be coadministered to the subject.

The expanded populations of antigen-specific immune effector cells of the present invention also find use in adoptive immunotherapy regimes and as vaccines.

Adoptive immunotherapies involve, for example, administering to a subject an effective amount of a substantially pure population of the expanded, educated, antigen-specific immune effector cells made by culturing naïve immune effector cells with hybrid cells, wherein the hybrid cells are antigen presenting cells (APCs) fused to cells that express one or more antigens and wherein the educated, antigen-specific immune effector cells are expanded at the expense of the hybrid cells and subsequently exposing the resulting educated, antigen-specific immune effector cells to an anti-CD3/CD28 antibody to further expand the population. Preferably, the APCs are DCs.

The cells can be autologous or allogeneic. For example, when the adoptive immunotherapy methods described herein are autologous, the hybrid cells are made using parental cells isolated from a single subject. The expanded population also employs T cells isolated from that subject. Finally, the expanded population of antigen-specific cells is administered to the same patient.

Alternatively, when the adoptive immunotherapy methods are allogeneic, cells from two or more patients are used to generate the hybrid cells, and stimulate production of the antigen-specific cells. For instance, cells from other healthy or diseased subjects can be used to generate antigen-specific cells in instances where it is not possible to obtain autologous T cells and/or dendritic cells from the subject providing the biopsy. The expanded population can be administered to any one of the subjects from whom cells were isolated, or to another subject entirely.

### Genetic Modifications

The methods of this invention are intended to encompass any method of gene transfer into either the hybrid cells or the antigen-specific population of cells derived using the hybrid cells as stimulators. Examples of genetic modifications includes, but are not limited to viral mediated gene transfer, liposome mediated transfer, transformation, transfection and transduction, e.g., viral mediated gene transfer such as the use of vectors based on DNA viruses such as adenovirus, adeno-associated virus and herpes virus, as well as retroviral based vectors. The methods are particularly suited for the integration of a nucleic acid contained in a vector or construct lacking a nuclear localizing element or sequence such that the nucleic acid remains in the cytoplasm. In these instances, the nucleic acid or therapeutic gene is able to enter the nucleus during M (mitosis) phase when the nuclear membrane breaks down and the nucleic acid or therapeutic gene gains access to the host cell chromosome. Genetic modification is performed *ex vivo* and the modified (*i*.*e*. transduced) cells are subsequently administered to the recipient. Thus, the invention encompasses treatment of diseases amenable to gene transfer into antigen-specific cells, by administering the gene *ex vivo* or *in vivo* by the methods disclosed herein.

The expanded population of antigen-specific cells can be genetically modified. In addition, the hybrid cells can also be genetically modified, for example, to supply particular secreted products including, but not limited to, hormones, enzymes, interferons, growth factors, or the like. By employing an appropriate regulatory initiation region, inducible production of the deficient protein can be achieved, so that production of the protein will parallel natural production, even though production will be in a different cell type from the cell type that normally produces such protein. It is also possible to insert a ribozyme, antisense or other message to inhibit particular gene products or susceptibility to diseases, particularly hematolymphotropic diseases.

Suitable expression and transfer vectors are known in the art.

Therapeutic genes that encode dominant inhibitory oligonucleotides and peptides as well as genes that encode regulatory proteins and oligonucleotides also are encompassed by this invention. Generally, gene therapy will involve the transfer of a single therapeutic gene although more than one gene may be necessary for the treatment of particular diseases. The therapeutic gene is a dominant inhibiting mutant of the wild-type immunosuppressive agent. Alternatively, the therapeutic gene could be a wild-type, copy of a defective gene or a functional homolog.

More than one gene can be administered per vector or alternatively, more than one gene can be delivered using several compatible vectors. Depending on the genetic defect, the therapeutic gene can include the regulatory and untranslated sequences. For gene therapy in human patients, the therapeutic gene will generally be of human origin although genes from other, closely related species that exhibit high homology and biologically identical or equivalent function in humans may be used, if the gene product does not induce an adverse immune reaction in the recipient. The therapeutic gene suitable for use in treatment will vary with the disease.

A marker gene can be included in the vector for the purpose of monitoring successful transduction and for selection of cells into which the DNA has been integrated, as against cells, which have not integrated the DNA construct. Various marker genes include, but are not limited to, antibiotic resistance markers, such as resistance to 0418 or hygromycin. Less conveniently, negative selection may be used, including, but not limited to, where the marker is the HSV-tk gene, which will make the cells sensitive to agents such as acyclovir and gancyclovir. Alternatively, selections could be accomplished by employment of a stable cell surface marker to select for transgene expressing cells by FACS sorting. The NeoR (neomycin /0418 resistance) gene is commonly used but any convenient marker gene whose sequences are not already present in the recipient cell, can be used.

The viral vector can be modified to incorporate chimeric envelope proteins or nonviral membrane proteins into retroviral particles to improve particle stability and expand the host range or to permit cell type-specific targeting during infection. The production of retroviral vectors that have altered host range is taught, for example, in WO 92/1 4829 and WO 93/14188. Retroviral vectors that can target specific cell types *in vivo* are also taught, for example, in Kasahara et al. (1994) Science 266:1373-1376. Kasahara et al. describe the construction of a Moloney leukemia virus (MoMLV) having a chimeric envelope protein consisting of human erythropoietin (EPO) fused with the viral envelope protein. This hybrid virus shows tissue tropism for human red blood progenitor cells that bear the receptor for EPO, and is therefore useful in gene therapy of sickle cell anemia and thalassemia. Retroviral vectors capable of specifically targeting infection of cells are preferred for *in vivo* gene therapy.

Expression of the transferred gene can be controlled in a variety of ways depending on the purpose of gene transfer and the desired effect. Thus, the introduced gene may be put under the control of a promoter that will cause the gene to be expressed constitutively, only under specific physiologic conditions, or in particular cell types.

Examples of promoters that may be used to cause expression of the introduced sequence in specific cell types include Granzyme A for expression in T-cells and NK cells, the CD34 promoter for expression in stem and progenitor cells, the CD8 promoter for expression in cytotoxic T-cells, and the CD11b promoter for expression in myeloid cells.

Inducible promoters may be used for gene expression under certain physiologic conditions. For example, an electrophile response element may be used to induce expression of a chemoresistance gene in response to electrophilic molecules. The therapeutic benefit may be further increased by targeting the gene product to the appropriate cellular location, for example the nucleus, by attaching the appropriate localizing sequences.

After viral transduction, the presence of the viral vector in the transduced cells or their progeny can be verified such as by PCR. PCR can be performed to detect the marker gene or other virally transduced sequences. Generally, periodic blood samples are taken and PCR conveniently performed using e.g. NeoR probes if the NeoR gene is used as marker. The presence of virally transduced sequences in bone marrow cells or mature hematopoietic cells is evidence of successful reconstitution by the transduced cells. PCR techniques and reagents are well known in the art, (*see, generally,* PCR PROTOCOLS, A GUIDE TO METHODS AND APPLICATIONS. Innis, Gelfand, Sninsky & White, eds. (Academic Press, Inc., San Diego, 1990)) and commercially available (Perkin-Elmer).

### Method of Screening Candidate Peptide and Peptides for Antigenic Activity

The CTL and HTL ("effector cells") described above can be used to identify antigens expressed by the non-dendritic cell partners of the fused cells used to generate the effector cells of the invention, by a number of methods used in the art. In brief, the effector cell-containing cell population is cultured together with a candidate peptide or polypeptide and either an appropriate target cell (where cytotoxicity is assayed) or antigen presenting cell (APC) (where cell proliferation, or cytokine production is assayed) and the relevant activity is determined. A peptide that induces effector activity will be an antigenic peptide, which is recognized by the effector cells. A polypeptide that induces effector activity will be an antigenic polypeptide, a peptide fragment of which is recognized by the effector cells.

Cytotoxic activity can be tested by a variety of methods known in the art (*e.g.,* ⁵¹Cr or lactate dehydogenase (LDH) release assays described in Examples I and III-V). Target cells can be any of a variety of cell types, *e.g.,* fibroblasts, lymphocytes, lectin (*e.g.,* phytohemagglutinin (PHA), concanavalin A (ConA), or lipopolysaccharide (LPS)) activated lymphocyte blasts, macrophages, monocytes, or tumor cell lines. The target cells should not naturally express the candidate antigens being tested for antigenic activity, though they could express them recombinantly. The target cells should, however, express at least one type of MHC class I molecule or MHC class II molecule (depending on the restriction of the relevant CTL), in common with the CTL. The target cells can endogenously express an appropriate MHC molecule or they can express transfected polynucleotides encoding such molecules. The chosen target cell population can be pulsed with the candidate peptide or polypeptide prior to the assay or the candidate peptide or polypeptide can be added to the assay vessel, *e.g.,* a microtiter plate well or a culture tube, together with the CTL and target cells. Alternatively, target cells transfected or transformed with an expression vector containing a sequence encoding the candidate peptide or polypeptide can be used. The CTL-containing cell population, the target cells, and the candidate peptide or polypeptide are cultured together for about 4 to about 24 hours. Lysis of the target cells is measured by, for example, release of ⁵¹Cr or LDH from the target cells. A peptide that elicits lysis of the target cells by the CTL is an antigenic peptide that is recognized by the CTL. A polypeptide that elicits lysis of the target cells by the CTL is an antigenic polypeptide, a peptide fragment of which is recognized by the CTL.

Candidate peptides or polypeptides can be tested for their ability to induce proliferative responses in both CTL and HTL. The effector cells are cultured together with a candidate peptide or polypeptide in the presence of APC expressing an appropriate MHC class I or class II molecule. Such APC can be B-lymphocytes, monocytes, macrophages, or dendritic cells, or whole PBMC. APC can also be immortalized cell lines derived from B-lymphocytes, monocytes, macrophages, or dendritic cells. The APC can endogenously express an appropriate MEC molecule or they can express a transfected expression vector encoding such a molecule. In all cases, the APC can, prior to the assay, be rendered non-proliferative by treatment with, *e*.*g*.,ionizing radiation or mitomycin-C. The effector cell-containing population is cultured with and without a candidate peptide or polypeptide and the cells' proliferative responses are measured by, *e.g.,* incorporation of [³H]-thymidine into their DNA.

As an alternative to measuring cell proliferation, cytokine production by the effector cells can be measured by procedures known to those in art. Cytokines include, without limitation, interleukin-2 (IL-2), IFN-, IL-4, IL-5, TNF-, interleukin-3 (IL-3), interleukin-6 (IL-6), interleukin-10 (IL-b), interleukin-12 (IL-12), interleukin-15 (IL-15) and transforming growth factor (TGF) and assays to measure them include, without limitation, ELISA, and bioassays in which cells responsive to the relevant cytokine are tested for responsiveness (*e.g*., proliferation) in the presence of a test sample. Alternatively, cytokine production by effector cells can be directly visualized by intracellular immunofluorescence staining and flow cytometry.

Choice of candidate peptides and polypeptides to be tested for antigenicity will depend on the non-dendritic cells that were used to make the fused cells. Where the non-dendritic cells are tumor cells, candidate polypeptides will be those expressed by the relevant tumor cells. They will preferably be those expressed at a significantly higher level in the tumor cells than in the normal cell equivalent of the tumor cells. Candidate peptides will be fragments of such polypeptides. Thus, for example, for melanoma cells, the candidate polypeptide could be tyrosinase or a member of the MART family of molecules; for colon cancer, carcinoembryonic antigen; for prostate cancer, prostate specific antigen; for breast or ovarian cancer, HER2/neu; for ovarian cancer, CA-125; or for most carcinomas, mucin-1 (MUC1).

On the other hand, where the non-dendritic cells used to generate the fused cells were infected cells or cells genetically engineered to express a pathogen-derived polypeptide, the candidate polypeptide will be one expressed by the appropriate infectious microorganism or that expressed by the transfected cells, respectively. Examples of such polypeptides include retroviral (*e.g.,* HIV or HTLV) membrane glycoproteins (*e.g.,* gpl60) or gag proteins, influenza virus neuraminidase or hemagglutinin, *Mycvbacterium tuberculvsis or leprae* proteins, or protozoan (*e.g., Plasmodium* or *Trypanosoma*) proteins. Polypeptides can also be from other microorganisms listed herein. Peptides to be tested can be, for example, a series of peptides representing various segments of a full-length polypeptide of interest, *e.g.,* peptides with overlapping sequences that, in tow, cover the whole sequence. Peptides to be tested can be any length. When testing MHC class I restricted responses of effector cells, they will preferably be 7-20 (*e.g.,* 8-12) amino acids in length. On the other hand, in MHC class II restricted responses, the peptides will preferably be 10-30 *(e.g.,* 12 - 25) amino acids in length.

Alternatively, a random library of peptides can be tested. By comparing the sequences of those eliciting positive responses in the appropriate effector cells to a protein sequence database, polypeptides containing the peptide sequence can be identified. Relevant polypeptides or the identified peptides themselves would be candidate therapeutic or vaccine agents for corresponding diseases (see below).

Polypeptides and peptides can be made by a variety of means known in the art. Smaller peptides (less than 50 amino acids long) can be conveniently synthesized by standard chemical means. In addition, both polypeptides and peptides can be produced by standard *in vitro* recombinant DNA techniques, and *in vivo* genetic recombination (*e.g.,* transgenesis), using nucleotide sequences encoding the appropriate polypeptides or peptides. Methods well known to those skilled in the art can be used to construct expression vectors containing relevant coding sequences and appropriate transcriptional/translational control signals. See, for example, the techniques described in Maniatis et al., Molecular Cloning: A Laboratory Manual [Cold Spring Harbor Laboratory, N.Y., 1989), and Ausubel et al., Current Protocols in Molecular Biology, [Green Publishing Associates and Wiley Interscience, N.Y., 1989).

A variety of host-expression vector systems can be used to express the peptides and polypeptides. Such host-expression systems represent vehicles by which the polypeptides of interest can be produced and subsequently purified, but also represent cells that can, when transformed or transfected with the appropriate nucleotide coding sequences, produce the relevant peptide or polypeptide *in situ.* These include, but are not limited to, microorganisms such as bacteria, *e*.*g*.,*E. coli* or B. *subtilis,* transformed with recombinant bacteriophage DNA, plasmid or cosmid DNA expression vectors containing peptide or polypeptide coding sequences; yeast, *e.g., Saccharomyces* or *Pichia,* transformed with recombinant yeast expression vectors containing the appropriate coding sequences; insect cell systems infected with recombinant virus expression vectors, *e*.*g*.,baculovirus; plant cell systems infected with recombinant virus expression vectors, *e.g.,* cauliflower mosaic virus (CaMV) or tobacco mosaic virus (TMV), or transformed with recombinant plasmid expression vectors, *e.g.,* Ti plasmids, containing the appropriate coding sequences; or mammalian cell systems, *e.g.,* COS, CHO, BHK, 293 or 3T3, harboring recombinant expression constructs containing promoters derived from the genome of mammalian cells, *e.g.,* metallothionein promoter, or from mammalian viruses, *e.g.,* the adenovirus late promoter or the vaccinia virus 7.5K promoter.

Peptides of the invention include those described above, but modified for *in vivo* use by the addition, at either or both the amino- and carboxyl-terminal ends, of a blocking agent to facilitate survival of the relevant peptide *in vivo.* This can be useful in those situations in which the peptide termini tend to be degraded by proteases prior to cellular or mitochondrial uptake. Such blocking agents can include, without limitation, additional related or unrelated peptide sequences that can be attached to the amino and/or carboxyl terminal residues of the peptide to be administered. This can be done either chemically during the synthesis of the peptide or by recombinant DNA technology by methods familiar to artisans of average skill. Alternatively, blocking agents such as pyroglutamic acid or other molecules known in the art can be attached to the amino and/or carboxyl terminal residues, or the amino group at the amino terminus or carboxyl group at the carboxyl terminus can be replaced with a different moiety. Likewise, the peptides can be covalently or noncovalently coupled to pharmaceutically acceptable "carrier" proteins prior to administration.

Also of interest are peptidomimetic compounds that are designed based upon the amino acid sequences of the peptides or polypeptides. Peptidomimetic compounds are synthetic compounds having a three-dimensional conformation (*i.e.,* a "peptide motif) that is substantially the same as the three-dimensional conformation of a selected peptide. The peptide motif provides the peptidomimetic compound with the ability to activate T cells in a manner qualitatively identical to that of the peptide or polypeptide from which the peptidomimetic was derived. Peptidomimetic compounds can have additional characteristics that enhance their therapeutic utility, such as increased cell permeability and prolonged biological half-life.

The peptidomimetics typically have a backbone that is partially or completely non-peptide, but with side groups that are identical to the side groups of the amino acid residues that occur in the peptide on which the peptidomimetic is based. Several types of chemical bonds, e.g., ester, thioester, thioamide, retroamide, reduced carbonyl, dimethylene and ketomethylene bonds, are known in the art to be generally useful substitutes for peptide bonds in the construction of protease-resistant peptidomimetics.

### Vaccines

The educated, expanded T cell populations and methods described herein can also be used to develop cell-based vaccines. Further provided by this invention are vaccines comprising antigen-specific immune effector cells according to the present invention. Still further provided by this invention is a vaccine comprising an antigen or a fragment thereof such as an epitope or sequence motif utilizing the antigen specific immune effector cells described herein. Methods of administering vaccines are known in the art and the vaccines may be combined with an acceptable pharmaceutical carrier. An effective amount of a cytokine and/or costimulatory molecule also can be administered along with the vaccine.

The polynucleotides, genes and encoded peptides and proteins according to the invention can be further cloned and expressed *in vitro* or *in vivo.* The proteins and polypeptides produced and isolated from the host cell expression systems are also within the scope of this invention. Expression and cloning vectors as well as host cells containing these polynucleotides and genes are claimed herein as well as methods of administering them to a subject in an effective amount. Peptides corresponding to these sequences can be generated by recombinant technology and they may be administered to a subject as a vaccine or alternatively, introduced into APC which in turn, are administered in an effective amount to a subject. The genes may be used to produce proteins which in turn may be used to pulse APC. The APC may in turn be used to expand immune effector cells such as CTLs. The pulsed APC and expanded effector cells can be used for immunotherapy by administering an effective amount of the composition to a subject.

The following examples are meant to illustrate, but not limit, the compositions and methods of the invention.

### Example 1: Generation of DC and tumor and DC/tumor fusion cell preparations

DCs were generated from adherent mononuclear cells isolated from leukopak collections obtained from normal donors. Peripheral blood mononuclear cells (PBMCs) were isolated from leukopaks from normal donors by Histopaque®-1077 density gradient centrifugation. PBMCs were suspended at 1x10⁶/ml in RPMI 1640 complete medium and plated in 5 ml aliquots in 6 well tissue culture plates and incubated for 2 h at 37°C in a humidified 5% CO₂ incubator. The monocyte enriched adherent fraction was cultured in RPMI 1640 complete medium containing GM-CSF (1000 U/ml) and IL-4 (1000 U/ml) for 5 days to generate immature DCs. The DC preparation underwent maturation by culturing the cells for an additional 48 h in the presence of TNFα (25 ηg/ml).

The renal cell carcinoma ("RCC") cell line, RCC 786, was maintained in RPMI 1640 culture media. Myeloid leukemia cells were obtained from bone marrow aspirates or peripheral blood collections obtained from patients with acute myeloid leukemia as per an institutionally approved protocol. Leukemia cells were isolated by ficoll density centrifugation and cultured with RPMI 1640 complete medium. DCs and tumor cells underwent phenotypic analysis by flow cytometry and immunohistochemistry as outlined below.

To generate fusion cells, tumor cells were mixed with DC preparations at ratios of 1:1 - 1:3 (dependent on cell yields) and washed 3 times in serum-free RPMI 1640 culture media. After the final wash, the cell pellet was resuspended in 1 ml of 50% polyethylene glycol (PEG) solution. After 2 minutes at room temperature, the PEG solution was progressively diluted and cells were washed twice with serum free media. The DC-tumor fusion cells were cultured in RPMI complete media in the presence of GM-CSF. DC/tumor fusions were quantified by determining the percentage of cells that expressed unique DC and tumor antigens by immunohistochemical analysis.

### Analysis of DC, tumor and fusion cell preparations by immunohistochemical analysis

DC, tumor, and fusion cell preparations underwent immunocytochemical analysis to assess for the presence of tumor associated antigens and DC associated costimulatory and maturation markers. RCC cells underwent staining with primary murine monoclonal antibodies (mAbs) against MUC1 (PharMingen, San Diego, CA), cytokeratin (Boehringer Mannheim, Indianapolis, IN), and CAM (Becton Dickson, San Jose, CA). Myeloid leukemia cells were stained for CD34, CD117, and MUC1. The Absence of DC markers, outlined below, was confirmed. *(See* Figure 1B). DC preparations underwent staining for HLA-DR, CD80, CD83 or CD86 (PharMingen) and an isotype-matched negative control for 60 min. *(See* Figure 1A). The cells were incubated with a biotinylated F(ab')2 fragment of horse anti-mouse IgG (Vector Laboratories) for 45 min, washed twice with PBS, and incubated for 30 min with ABC (avidin-biotin complex) reagent solutions followed by AEC (3 amino-9-ethyl carbazole) solution (Vector Laboratories). In the fusion cell preparations, detection of tumor associated antigens with the ABC reagents was followed by staining for DC associated markers with the ABC-AP (alkaline phosphatase) kit (Vector Laboratories). Slides were washed, fixed in 2% paraformaldehyde, and analyzed using an Olympus AX70 microscope. Fusion cells were quantified by determining the percentage of cells that coexpressed unique DC and tumor antigens. *(See* Figure 1C).

### Flow cytometric analysis

DC, tumor, and fusion cell preparations also underwent flow cytometric analysis to assess for expression of the antigens outlined above. Cells were incubated with the indicated primary mAb or a matching isotype control for 30 min at 4°C. Bound primary mAbs were detected with a secondary affinity purified FITC-conjugated goat anti-mouse IgG (Chemicon Intl, Temecula, CA) followed by fixation in 2% paraformaldehyde. For bidimensional flow cytometry, cells were incubated with antibody directed against tumor associated antigens (RCC-MUC1, CAM or cytokeratin, AML-CD34, CD117, or MUC1), FITC-conjugated secondary antibody and antibody directed against DR or CD86 conjugated with PE. Analysis was performed on the FACS Calibur flow cytometer (Becton Dickinson) using CellQuest software (Becton Dickinson).

### Example 2: T cell stimulation and expansion with DC/tumor fusions and/or anti-CD3/CD28:

Nonadherent PBMCs were isolated from the leukopak collection used for DC generation and cultured at a density of 1x10⁶/ml in RPMI complete media in the presence of 10 U/ml IL-2. T cells were isolated by nylon wool separation. T cells were exposed to the immobilized monoclonal antibodies, anti-CD3 (clone-UCHT1; Pharmingen) and anti-CD28 (clone-CD28.2; Pharmingen; CD3i/CD28i). Twenty-four-well non-tissue culture-treated plates (Falcon, Fisher) were coated with each of the antibodies (1 uglml in PBS) and left overnight at 37°C. T cells were: 1) cultured on the anti-CD3/CD28 coated plates for 48 h; 2) cocultured with fusion cells for 5 days at a fusion to T cell ratio of 1:10; 3) cocultured with fusion cells and followed by anti-CD3/CD28 coated plates for 48 h; or 4) cultured with anti-CD3/CD28 for 48 h followed by stimulation with fusions for 5 d. Following stimulation, T cells underwent phenotypic analysis as outlined below.

### Proliferation of stimulated T cell populations

Following stimulation, T cells were harvested and proliferation was determined by incorporation of [³H]-Thymidine (1µCi/well; 37kBq; NEN-DuPont, Boston, MA) added to each well 18 h before the end of the culture period. Thereafter, the cells were harvested onto glass fiber filter paper (Wallac Oy, Turku, Finland) using an automated TOMTEC harvester (Mach II, Hamden CT), dried, placed and sealed in BetaPlate sample bag (Wallac) with 10 mls of ScintiVerse®(Fisher Scientific, Fair Lawn, NJ). Cell bound radioactivity was counted in a liquid scintillation counter (Wallac, 1205 Betaplate™). (*See* Figure 2). Data are expressed as Stimulation Index ("SI"). The SI was determined by calculating the ratio of [³H]-Thymidine incorporation (mean of triplicates) over background [³H]-Thymidine incorporation (mean of triplicates) of the unstimulated T cell population. T cells did not demonstrate significant proliferation following exposure to DC/RCC fusions or anti-CD3/CD28 alone with SI of 0.9 and 1.0, respectively (N=9). In contrast, stimulation with DC/RCC fusions followed by exposure to CD3/CD28 resulted in a dramatic and synergistic increase in T cell proliferation with an SI of 13.2 (p=0.03 compared to stimulation with fusions alone). (*See* Figure 2A). Of note, exposure to anti-CD3/CD28 prior to fusion cell stimulation did not induce T cell proliferation (SI 1.0).

T cells stimulated by fusion cells, anti-CD3/CD28, or sequential stimulation with fusions followed by anti-CD3/CD28 underwent phenotypic analysis by multichannel flow cytometry to assess for the presence of naïve (CD45 RA), memory (CD45RO), activated (CD69, IFNγ) or regulatory (Foxp3, IL-10) T cells. The cells were washed and incubated with blocking buffer (10% human IgG; Sigma) and incubated with FITC conjugated CD4 or CD8 and PE conjugated CD45RA or CD45RO. T cell preparations were stained for FITC conjugated CD4, cytochrome conjugated CD25, and PE conjugated CD69 (PharMingen). Alternatively, cells were stained for CD4/CD25 and then permeabilized by incubation in Cytofix/Cytoperm plus™ (containing formaldehyde and saponin) (PharMingen). Cells were then incubated with PE conjugated anti-human IFNγ, IL-10, or Foxp3 (Caltag, Burlingame, CA) or a matched isotype control antibody, washed in Perm/Wash™ solution, fixed in 2% paraformaldehyde and analyzed by flow cytometry using FACScan (Becton Dickinson).

The effect of combined stimulation with DC/RCC fusions and anti-CD3/CD28 on the relative expansion of naive and memory cells was subsequently studied. (*See* Figure 2B). In four serial studies, unstimulated T cells demonstrated CD45RO/CDRA ratio of 0.9, which represent mean levels of 21% and 24% of the total CD4+ T cell population, respectively. Stimulation with DC/RCC fusions did not alter this ratio with mean levels of 17% and 22%, of CD45RA and CD45RO, respectively (ratio 0.8). Exposure of the T cells to anti-CD3/CD28 resulted in the relative suppression of CD45RA cells which represented 9% of the T cell population while the CD45RO+ cells were largely unchanged (24%).

In contrast, sequential stimulation with DC/RCC fusions and anti-CD3/CD28 resulted in the expansion of CD45RO+ cells which represented 40% of the T cells with a more modest decrease in the CD45RA levels (CD45RO/CD45RA ratio of 2.9). Initial exposure to anti-CD3/CD28 followed by exposure to DC/RCC fusions did not result in the expansion of the CD45RO populations (mean level of 23%) but a decrease in mean levels af CD45RA cells was observed. These data suggest that sequential stimulation with DC/RCC fusions and anti-CD3/CD28 uniquely expands memory effector cells.

### Stimulation of activated as compared to regulatory T cells

A determination of whether combined stimulation with DC/RCC fusions and anti-CD3/CD28 resulted in the expansion of activated as compared to regulatory T cells was also made. These two populations of T cells co-express CD4 and CD25. Activated T cells characteristically express high levels of CD69, while Foxp3 has been shown to be a relatively specific marker for regulatory T cells. The phenotypic characteristics of T cells stimulated by DC/RCC fusions, anti-CD3/CD28 or sequential stimulation with these agents was examined. (*See* Figure 3). In 11 experiments, a modest increase in CD4+/CD25+ was observed following stimulation with DC/RCC fusions alone. Mean percentage of CD4+/CD25+ cells of the total T cell population increased from 2.8% to 6.4%. Similarly, following stimulation of T cells with anti-CD3/CD28 alone, 7.8% of the CD4⁺ cells demonstrated co-expression of CD4 and CD25.

In contrast, a marked increase in the mean percentage of CD4+/CD25+ cells was observed following sequential stimulation with DC/RCC fusions and anti-CD3/CD28 reaching a level of 25.3% of the total T cell population (p=0.001, 0.02, and 0.002 as compared to unstimulated T cells, T cells stimulated by fusions; and T cells stimulated by anti-CD3/CD28 alone, respectively). The sequence of exposure was crucial in that stimulation by anti-CD3/CD28 followed by fusion cells resulted in only 10% of cells expressing CD4+/CD25+ (p=0.008 compared to stimulation with fusion followed by anti-CD3/CD28).

To further define the nature of the CD4+/CD25+ cells, multichannel flow cytometric analysis was performed to determine whether the CD4/CD25 population expressed markers of activation or suppression. (*See* Figure 3). CD4+/CD25+ T cells were isolated by FACS gating and expression of CD69 and Foxp3 was determined. Results were presented as the percentage of activated or regulatory T cells out of the total CD4/CD25+ T cell population. Stimulation of T cells with DC/RCC fusions alone or anti-CD3/CD28 alone resulted in a 5 and 6 fold increase, respectively in the percentage of activated T cells, defined as CD4+/CD25+/CD69+ cells. Remarkably, a 42 fold increase in the percentage of CD4+/CD25+/CD69+ cells was observed following sequential stimulation with fusion cells followed by anti-CD3/CD28, thereby demonstrating a statistically significant increase as compared to anti-CD3/CD28 alone (6 fold increase p=0.01), fusion cells alone (5 fold increase, p=0.05) or after anti CD3/CD28 expansion followed by stimulation with fusions (9 fold increase, p=0.02).

Similarly, the effect of combined stimulation of DC/RCC fusions and anti-CD3/CD28 on expansion of regulatory T cells as defined by cells co-expressing of CD4, CD25, and FOXP3 was also examined. (*See* Figure 3). In nine experiments, the combination of stimulation with DC/tumor fusion vaccine followed by expansion with anti CD3/CD28 resulted in a 15 fold expansion of regulatory T cells which was statistically greater than that observed following stimulation with fusions alone (1.9 fold p=0.008), anti-CD3/CD28 alone (1.7 fold p=0.004), or sequential stimulation with anti-CD3/CD28 and fusions (3.4 fold p=0.03). These data suggest that sequential stimulation with DC/RCC fusions and anti-CD3/CD28 synergistically induces T cell proliferation and expansion of activated T cells far in excess to that observed with either DC/RCC or anti-CD3/CD28 alone. In addition, this result was uniquely observed when T cells were first stimulated with the DC/RCC fusions suggesting that DC mediated antigen specific stimulation was crucial prior to the antigen independent expansion created by ligation of the anti-CD3/CD28 complex. Of note, combined stimulation with DC/RCC fusions and anti-CD3/CD28 also increased the percentage of regulatory T cells but to a lesser degree.

### Assessment of tumor specific immune responses by binding to the MUC1 tetramer and cytolytic capacity by granzyme B expression

Antigen specific MUC1+CD8+ T cells were identified using phycoerythrin (PE) labeled HLA-A*0201⁺iTAg™ MHC class I human tetramer complexes composed of four HLA MHC class 1 molecules each bound to MUC1-specific epitopes M 1.2 (MUCl₁₂₋₂₀) LLLLTVLTV (SEQ ID NO:1) (Beckman Coulter, Fullerton, CA). A control PE-labeled tetramer was used in parallel. T cells stimulated by anti-CD3/CD28, fusions, or sequential exposure to anti-CD3/CD28 and fusions were incubated with the MUC1 or control tetramer and then stained with FITC-conjugated CD8 antibody. Cells were washed and analyzed by bi-dimensional FACS analysis. Cytolytic capacity of the stimulated T cell populations was assessed by staining with FITC conjugated CD8 and PE conjugated granzymeB. A total of 3x10⁵ events were collected for final analysis. Similarly, non-adherent unstimulated cells were analyzed in parallel.

### Functional Characteristics of Stimulated T cell populations

To further characterize the functional characteristics of the T cell populations, the intracellular expression of TH-1 and TH-2 cytokines by T cells that had been stimulated with fusion cells, anti-CD3/CD28, or their combination was identified. In 8 serial studies, intracellular expression of IFNγ was observed in 0.5% of the unstimulated CD4+ T cell population. Following stimulation with anti-CD3/CD28 or DC/RCC fusions alone, the mean percentage of IFNγ expressing T cells rose to 1.7% and 1.8%, respectively. In contrast, sequential stimulation with DC/RCC fusions and anti-CD3/CD28 resulted in statistically significant increase in mean levels of IFNγ expressing cells (4.7%, p= 0.05 as compared to stimulation with anti-CD3/CD28 or fusions, respectively) representing a 10.5 fold increase as compared to unstimulated T cells (p=0.008) (Figure 16). Stimulation of T cells with anti-CD3/CD28 alone resulted in an increase of the percent of CD4+ T cells demonstrating intracellular expression of IL-4 from 1.0% to 2.4%. In contrast, exposure to DC/RCC fusions alone or sequential stimulation with DC/RCC fusions and anti-CD3/CD28 did not result in an increase in IL-4 expression (0.9% and 0.6%, respectively). Mean intracellular expression of IL-10 increased from 0.9 to 3.4% following stimulation with DC/RCC fusions and anti-CD3/CD28. In comparison, no increase in IL-10 expression was observed following stimulation with DC/RCC fusions alone. These data suggest that sequential stimulation with DC/RCC fusions induces of the expansion of activated effector cells expressing IFNγ with a relatively more modest increase in T cells expressing IL-10.

### Expansion of tumor reactive T cells with cytolytic capacity

To determine if sequential stimulation with DC/RCC fusion and anti-CD3/CD28 resulted in the selective expansion of tumor reactive lymphocytes, whether T cells specific for the tumor associated antigen, MUC1, were increased following expansion was examined (Figure 17). DCs and T cells were isolated from an HLA -A2.1 donor for this analysis. Following stimulation with anti-CD3/CD28 alone, only 0.93% of the CD8 population bound the MUC1 tetramer. In contrast, coculture with DC/RCC fusions resulted in an increase in MUC1 tetramer+ cells (2.3%). Of note, sequential stimulation with DC/RCC fusions followed by anti-CD3/CD28 resulted in a dramatic increase in MUC1 tetramer+ cells (17.3%, p=0.02 and 0.004 as compared to stimulation with fusions or antiCD3/CD28 alone, respectively). In contrast, nonspecific stimulation with anti-CD3/CD28 followed by coculture with fusions did not induce the expansion of MUC1 tetramer + cells (0.19%). These data suggest that initial exposure to an antigen specific stimulus with the DC/RCC was crucial for the subsequent expansion of tumor reactive T cells using anti-CD3/CD28.

Subsequently, whether T cells stimulated by DC/RCC fusions and anti-CD3/CD28 demonstrate cytolytic capacity as evidenced by expression of granzyme B was examined. Expression of granzyme is upregulated in activated cytolytic CD8+ T cells who demonstrate perforin mediated killing of target cells. Stimulation with DC/RCC fusions resulted in a 5.6 fold increase in CD8+ T cells expressing granzyme (Figure 18). Exposure to anti-CD3/CD28 resulted in only a 2 fold increase in granzyme+ cells. However, sequential stimulation with DC/RCC fusions and anti-CD3/CD28 induced a 21-fold expansion of granzyme + cells. Primary exposure to anti-CD3/CD28 followed by DC/RCC fusions did not result in further expansion of granzyme+ cells as compared to that observed following stimulation with anti-CD3/CD28 alone. These data suggest that sequential stimulation with DC/RCC fusions and anti-CD3/CD28 is uniquely effective in expanding functionally potent cytotoxic T lymphocytes.

### Stimulation with DC/AML fusions and anti-CD3/CD28

Subsequently, the phenotypic characteristics of T cells undergoing sequential stimulation with DC/tumor fusions using patient derived acute myeloid leukemia samples and anti-CD3/CD28 was examined. Myeloid leukemia cells were obtained from peripheral blood or bone marrow in patients with high levels of circulating disease and fused with DCs generated from normal leukopak collections. DC/AML fusions were quantified as determined by the percentage of cells that coexpressed antigens unique to the DC (CD86) and myeloid leukemia (CD117-ckit ligand, CD34, and/or MUC1) (Figure 19). Mean fusion efficiency was 28% of the total cell population. DC/AML fusions induced modest autologous T cell proliferation with an SI of 3.3 with memory effector cells (CD45RO+) comprising 10% of the total T cell population. Sequential stimulation with DC/AML fusions followed by anti-CD3/CD28 resulted in a statistically significant rise in T cell proliferation (SI 8.2) of which 39% expressed CD45RO (Figure 20). Similarly, a rise in CD4+/CD25+ cells was observed following sequential stimulation with DC/AML fusions followed by anti-CD3/CD28 (9.3% vs. 2.7% following stimulation with DC/AML fusions alone). In addition, an increased percentage of CD4+/CD25+ cells expressed IFNγ when exposed to anti-CD3/CD28 following coculture with fusion cells. A rise in the percent of Foxp3+ cells was also observed but this did not meet statistical significance. Sequential stimulation with DC/AML fusions and anti-CD3/CD28 induced granzyme B expression in 13% of the CD8+ population. In contrast, stimulation with fusion cells alone or anti-CD3/CD28 followed by fusions resulted in granzyme B expression in 2.5% and 2.7% of the CD8+ cells. Similar to the results observed in the RCC model, these data demonstrate that stimulation with DC/AML fusions followed by exposure anti-CD3/CD28 resulted in significant increase in activated T cells with cytolytic capacity.

### Example 3: Fusions of Dendritic Cells with Breast Carcinoma

### Generation of monocyte derived DCs

Peripheral blood mononuclear cells (PBMCs) were isolated from leukopaks from normal donors and from peripheral venous blood collected from patients with breast cancer as per an institutionally approved protocol. Samples underwent Histopaque®-1077 (Sigma) density gradient centrifugation and were plated in tissue culture flasks (Becton Dickinson, Franklin Lakes, NJ) in RPMI 1640 culture media containing 2 mM L-glutamine (Mediatech, Herndon, VA) and supplemented with heat inactivated 10% human AB male serum (Sigma, St. Louis, MO), 100 U/ml penicillin and 100 µg/ml streptomycin (Mediatech) (complete medium) for 2 h at 37°C in a humidified 5% CO₂ incubator. The monocytes-enriched adherent fraction was cultured in complete medium containing GM-CSF (1000 U/ml) (Berlex, Wayne/Montville, NJ) and IL-4 (1000 U/ml) (R&D Systems, Minneapolis, MN) for 5 days to generate immature DCs. A fraction of the DC preparation underwent further maturation by culturing the cells for an additional 48 h in the presence of TNFα (25 ηg/ml) (R&D Systems) or the combination of cytokines consisting of TNFα (25 ηg/ml), IL-1β (10 ηg/ml), IL-6 (1000 U/ml) (R&D Systems) and PGE₂ (1µg/ml) (Calbiochem-San Diego, CA). Maturation was effectively induced by exposure to TNFα for 48-96 hours resulting in increased expression of CD80 and CD83. (*See* Figure 4A). In 15 successive experiments, both immature and mature DC preparations strongly expressed the costimulatory molecule, CD86, (75% and 84%, respectively) and demonstrated low levels of expression of CD 14. (*See* Figure 4B). However, mature DC demonstrated a statistically significant increase in mean expression of CD80 (20% vs. 9%, p = 0.05) and CD83 (31% vs. 7% p=0.0003). As a measure of their functional capacity as antigen presenting cells, DC preparations were examined for their ability to stimulate allogeneic T cell proliferation. In successive studies, mature as compared to immature DCs stimulated higher levels of allogeneic T cell proliferation.

### Isolation and culture of T cells

T cells were isolated from the nonadherent PBMC fraction using a T-cell enrichment column (R & D Systems) or nylon wool column (Polysciences, Warrington, PA). Purity of T cells by both methods was >90% as determined by FACS analysis of CD3 surface expression. T cells were classified as allogeneic when derived from a third party donor and autologous when derived from the same donor from whom the DC fusion partner was derived.

### Isolation and culture of tumor cells

Primary breast carcinoma cells were obtained from malignant effusions or resected tumor lesions as per an institutionally approved protocol. Human breast carcinoma cell lines MCF-7 and ZR75-1 were purchased from ATCC (Manassas, VA). All tumor cell lines were maintained in DMEM (high glucose) or RPMI 1640 supplemented with 2 mM L-glutamine, 100 U/ml penicillin, 100 µg/ml streptomycin and 10% heat-inactivated fetal bovine serum (HyClone, Logan, UT).

### Preparation of DC/breast carcinoma fusion cells

Tumor cells were mixed with immature or mature DC preparations at ratios of 1:3 - 1:10 (dependent on cell yields) and washed 3 times in serum-free prewarmed RPMI 1640 culture media. The cell pellet was resuspended in 50% polyethylene glycol (PEG) solution (molecular weight: 1450)/DMSO solution (Sigma-Aldrich, St. Louis, MO). After 3 minutes at room temperature, the PEG solution was progressively diluted with prewarmed serum-free RPMI medium and washed twice with serum free media. The fusion preparation was cultured for 5-7 days in 5% CO2 at 37° C in complete medium with GM-CSF (500 IU/ml).

### Characterization of DC, breast carcinoma, and DC/breast carcinoma fusion preparations by flow cytometry

The phenotypic characteristics of the fusion cell populations generated with immature and mature DC was examined. Immature and mature DC populations were fused with primary patient-derived breast cancer cells or the MCF-7 human breast carcinoma cell line by co-culture with PEG. DCs and breast carcinoma cells were incubated with primary mouse anti-human monoclonal antibodies directed against HLA-DR, CD11c, CD 14, CD80, CD86, CD83, CD40, CD54, MUC-1, cytokeratin and matching isotype controls (Pharmingen-San Diego, CA), washed, and cultured with FITC-conjugated goat anti-mouse IgG₁ (Chemicon International-Temecula, CA). Cells were fixed in 2% paraformaldehyde (Sigma) and underwent flow cytometric analysis using FACScan (Becton Dickinson, San Jose, CA) and CellQuest Pro software© (Becton Dickinson). DC/breast carcinoma fusions preparations were subjected to dual staining to quantify the percentage of cells that co-expressed unique DC (CD11c-Cychrome) and tumor antigens (MUC-1 or cytokeratin-FITC). Fusion cells were quantified by determining the percentage of cells that co-expressed unique tumor (MUC-1 and/or cytokeratin) and DC (CD11c) antigens.

Approximately 1.2 x 10⁴ cells were spun onto slides (Cytospin®, Shandon Lipshaw, Pittsburgh, PA), allowed to dry, and fixed with acetone. The slides were incubated with primary mouse anti-human mAbs MUC-1 and cytokeratin and an isotype-matched negative control at room temperature for 1 hour, washed, incubated with 1:100 biotinylated F(ab')2 fragment of horse anti-mouse IgG (Vector Laboratories, Burlingame, CA), washed, and incubated for 30 min with ABC (avidin-biotin complex) reagent solutions (Vector Laboratories) followed by AEC (3 amino-9-ethyl carbazole) solution (Vector Laboratories). Cells were then stained for HLA-DR, CD86, or CD83 with the ABC-AP (alkaline phosphatase) kit (Vector Laboratories). Slides were washed, fixed in 2% paraformaldehyde (Sigma), and analyzed using an Olympus AX70 microscope (Melville, NY).

Fusion cells were isolated by FACS gating and underwent staining with PE-conjugated mouse anti-human antibodies directed against CCR7, CD80, CD86 or CD83. The percentage of fusion cells expressing these markers was determined by multichannel flow cytometric analysis. Alternatively, an aliquot of fusion cells were pulsed with GolgiStop (1 µg/ml; Pharmingen), permeabilized by incubation in Cytofix/Cytoperm plus™ (containing formaldehyde and saponin) (Pharmingen) and washed in Perm/Wash™ solution (Pharmingen). The cells were then incubated with PE-conjugated anti-human IL-10 or IL-12 (Caltag Laboratories-Burlingame, CA) or a matched isotype control antibody for 30 min, washed twice in Perm/Wash™ solution and fixed in 2% paraformaldehyde (Sigma). A minimum of 1x10⁴ events were acquired for analysis.

In 12 successive studies, equivalent mean fusion efficiencies were observed following fusion of tumor cells with mature (11% ± 1.6 SEM) and immature (7% ± 1.2 SEM) DC. Fusion cells were isolated by FACS gating around cells that co-expressed DC and tumor derived antigens. In these studies, expression of CD86 was uniformly observed in both immature DC/breast carcinoma (89%) and mature DC/breast carcinoma (82%) fusion populations. (*See* Figures 5A, B). The maturation marker, CD83, was seen in 46% and 51% of immature and mature fusion cell populations, respectively (p=0.5, NS). (*See* Figure 5A and 5B). Immunocytochemical staining demonstrated prominent expression of DR, CD86 and CD83 by the immature DC/tumor fusions. (*See* Figures 5C-5F). These studies demonstrate fusion of DC and breast carcinoma cells results in phenotypic characteristics consistent with maturation and activation and was not associated with inhibition of DC differentiation.

### Expression of IL-12 and IL-10 by immature and mature DC/tumor fusions

As a measure of their potency as antigen presenting cells and their capacity to stimulate TH1 responses, the expression of IL-12 and IL-10 by the fusion cell populations was examined. (*See* Figures 6A and 6B). Fusion cells were isolated by FACS gating of cells that co-expressed DC and tumor derived antigens. Fusion cells generated with mature and immature DC and breast carcinoma were compared in 12 separate experiments. The mean percentage of fusion cells that express IL-12 and IL-10 did not differ between the fusion cell populations. IL-12 was expressed by approximately 40% (± 6.7 SEM) and 49% (± 6.3 SEM) (p=0.35, NS) and IL-10 by approximately 36.3% (± 6.4 SEM) and 40% (± 6.4 SEM; n=11) (p= NS) of the immature and mature DC/breast carcinoma fusions, respectively.

### Expression of CCR7 by immature and mature DC/tumor fusions

The chemokine receptor, CCR7, directs cell migration to sites of T cell traffic in the draining lymph nodes and is characteristically expressed by DCs that are undergoing maturation and activation. As a measure of their migratory capacity, expression of CCR7 was determined for fusions generated with immature and mature DC. (*See* Figure 6C). CCR7 was prominently expressed on both immature and mature fusion populations suggesting that tumor-DC fusion resulted in the expression of a mature and activated phenotype. In 12 experiments, mean CCR7 expression was observed in 33% (± 9 SEM) and 38% (± 7.3 SEM; n=11) of the immature and mature DC/breast cancer fusions, respectively.

### Example 4: Stimulation of allogeneic T cell proliferation by DC, tumor, and DC/breast carcinoma fusions

To assess their capacity to stimulate allogeneic T cell proliferation, immature and mature DCs and DC/breast carcinoma fusion cell preparations were cocultured with allogeneic normal donor derived T cells at a ratio of 1:10, 1:30, 1:100, 1:300 and 1:1000 in 96 well U-bottom culture plates (Costar, Cambridge, MA) for 5 days at 37°C and 5% CO₂. T-cell proliferation was determined by incorporation of [³H]-Thymidine (1µCi/well; 37kBq; NEN-DuPont, Boston, MA) added to each well 18 hrs before the end of the culture period. Thereafter, the cells were harvested onto glass fiber filter paper (Wallac Oy, Turku, Finland) using an automated TOMTEC harvester (Mach II, Hamden CT), dried, placed and sealed in BetaPlate sample bag (Wallac) with 10 mls of ScintiVerse®(Fisher Scientific, Fair Lawn, NJ). Cell bound radioactivity was counted in a liquid scintillation counter (Wallac, 1205 Betaplate™). Data are expressed as Stimulation Index (SI). The SI was determined by calculating the ratio of [³H]-Thymidine incorporation (mean of triplicates) over background [³H]-Thymidine incorporation (mean of triplicates) of the unstimulated T cell population.

### Cytokine expression by T cells stimulated by immature and mature DC/breast carcinoma fusions

The profile of secreted cytokines by T cells cultured with immature and mature DC/breast carcinoma fusions was determined using the cytometric bead array (CBA) kits (Becton Dickinson). Supernatants from unstimulated T cells or cells exposed to unfused DC and breast carcinoma served as controls. Supernatants were collected before cell harvest and frozen at -80C. Concentrations of IL-2, IL-4, IL-5, IL-10, IFNγ, TNFα, IL-12, IL-6, IL-1β and IL-8 were quantified using an inflammatory CBA kit as per standard protocol. Briefly, the kits provided a mixture of six microbead populations with distinct fluorescent intensities (FL-3) that are precoated with capture antibodies specific for each cytokine. Culture supernatant or the provided standardized cytokine preparations were added to the premixed microbeads and then cultured with secondary PE conjugated antibodies. Individual cytokine concentrations were indicated by their fluorescent intensities (FL-2) and then computed using the standard reference curve of Cellquest and CBA software (BD Pharmingen). Interassay reproducibility was assessed by using two replicate samples of three different levels of the human standards in three separate experiments.

The functional capabilities of mature DC/tumor fusion populations as compared to immature DC/tumor fusion populations were analyzed by comparing their abilities to stimulate T cell proliferation and cytokine production. Fusion cell populations were cocultured with autologous T cells for 5 days and proliferation was determined by measuring uptake of tritiated thymidine after overnight pulsing. (*See* Figure 6D). Proliferation was measured as the T cell stimulation index (SI) (Stimulated T cells/Unstimulated T cells). Both immature and mature DC/breast cancer fusions stimulated autologous T cell proliferation with SI of 3.3 (± 1.4 SEM; n=6) and 3.5 (± 1.4 SEM; n=6), respectively. Cytokine secretion of stimulated T cell populations was quantified using the BD cytometrix array bead system (BD Biosciences). (*See* Figure 7). Mean levels of IFNγ following stimulation with immature and mature DC/breast cancer fusions was 2188 and 2252 pg/ml, respectively. These levels were significantly greater than that seen with T cells cultured with unfused autologous DC (685 pg/ml). Fusion cell preparations did not induce a statistically significant increase in IL-12, IL-4, IL-10, IL-2 and TNFα production in the supernatant.

### CTL response following stimulation with immature

### and mature DC/breast carcinoma fusions

DC/breast carcinoma fusion cell preparations generated with immature and mature DCs were cocultured with autologous T cells at a ratio of 1:10 for 7-10 days. DC/breast carcinoma fusions generated with DC autologous to T cell effectors were used as target cells in a standard 5-h ⁵¹Cr-release assay. Target cells (2x10⁴ cells/well) were incubated with ⁵¹Chromium (NEN-DuPont) for 1 h at 37°C followed by repeated washes. ⁵¹Cr release was quantified following 5 hour coculture of effector and target cell populations. Percentage cytotoxicity was calculated using mean of triplicates by a standard assay as follows: % specific cytotoxicity = [(sample counts - spontaneous counts)/(maximum counts - spontaneous counts)] x 100. Spontaneous release was less than 25% of the maximum ⁵¹Cr uptake.

### Stimulation of tumor specific CTL responses and MUC-1 specific responses.

Both immature and mature DC/tumor populations were capable of generating significant levels of target specific killing, as demonstrated by the lysis of autologous tumor or semi-autologous fusion targets. In 10 separate experiments, CTL activity did not differ between the fusion populations. Mean CTL lysis for effector:T cell ratio of 30:1 was 27% for T cell stimulated with mature and immature DC/breast cancer fusions. *(See* Figure 8A). To assess the capacity of the fusion vaccine to stimulate T cell responses directed against a specific tumor antigen, HLA-A2.1+ T cells stimulation by DC/breast carcinoma fusions recognized MUC1 was assessed. Selective expansion of CD8+ T cells binding the MUC-1 tetramer was observed following fusion cell stimulation. *(See* Figure 8B). In summary, DC/breast carcinoma fusions demonstrate an activated phenotype with strong expression of costimulatory molecules, stimulatory cytokines, and chemokine receptors enabling them to migrate to sites of T activation. DC/breast carcinoma fusions stimulate anti-tumor CTL responses including the expansion of T cells targeting defined tumor antigens.

### Tetramer staining

Antigen specific MUC1+CD8+ T cells were identified by using phycoerythrin (PE) labeled HLA-A*0201⁺iTAg™ MHC class I human tetramer complexes composed of four HLA MHC class 1 molecules each bound to MUC1-specific epitopes M1.2 (MUC₁₂₋₂₀) LLLLTVLTV (SEQ ID NO: 1) (Beckman Coulter, Fullerton, CA). A control PE-labeled tetramer was used in parallel. Non-adherent cells were cocultured with DC/breast carcinoma fusion cells for 5 days, harvested, incubated with the MUC1 or control tetramer and then stained with FITC-conjugated CD8 antibody. Cells were washed and analyzed by bidimensional FACS analysis. A total of 3x10⁵ events were collected for final analysis. Similarly, non-adherent unstimulated cells were analyzed in parallel.

### Analysis of regulatory and activated T cell response to stimulation with DC/breast carcinoma fusions

Autologous and allogeneic T cell preparations were cocultured with mature DC/breast carcinoma fusions for 5 days at a 10:1 ratio. The cell preparations were incubated with FITC conjugated anti-CD4, cytochrome conjugated anti-CD25, and PE-conjugated anti-CD69, anti-GITR, or anti-CTLA-4. Alternatively, cells were permeabilized and cultured with PE conjugated antibody directed against IFNγ, IL-10, IL-4 or FOXP3. Cells were subsequently analyzed by multichannel flow cytometry. In some studies, CD4+ T cells were isolated by magnetic microbead isolation (Miltenyi Biotec), and the resultant population were subjected to a two staining procedure with CD25 antibody and the indicated marker.

Having characterized DC/breast carcinoma fusions as potent antigen presenting cells with the capacity to elicit activated T cell responses, the ability of fusion cells to also stimulate inhibitory elements that would suppress vaccine response was examined. Specifically, whether DC/tumor fusions induce the expansion of regulatory as compared to activated T cells was examined. While both activated memory effector cells and regulatory T cells coexpress CD4 and CD25, regulatory T cells may be differentiated by their relatively high level of CD25 expression and the presence of other markers such as GITR, CTLA-4, and Foxp3. In contrast, CD69 is characteristically expressed by activated T cells. Mature DCs were fused to a human breast carcinoma cell line (MCF7 or ZR75-1) and cocultured with autologous or allogeneic T cells for 5 days. CD4/CD25+ cells were quantified by flow cytometric analysis and further characterized with respect to expression of cell surface markers and cytokine profile. CD4+ T cells were positively selected from this population using the CD4+ magnetic beads. FACS analysis of the resultant CD4+ T cells demonstrated a purity of greater than 97%.

In a series of 13 separate experiments, stimulation with DC/breast carcinoma fusions did not result in an increase in the percentage of CD4+CD25+ T cells (7% ± 1.3 SEM as compared to unstimulated T cells 6.9% ± 1.1 SEM). (*See* Figure 9A). However, coculture of fusion cells and autologous T cells resulted in a 6.3 fold increase in CD4+CD25+ T cells that expressed CD69, (4.7%-unstimulated T cells; 29.5-fusion stimulated cells, N=5; p=0.01) consistent with an activated phenotype. Stimulation with mature DC/breast carcinoma fusions also resulted in 9 and 5.2 fold increase in CD4+CD25+ T cells that expressed GITR and CTLA-4, respectively. These findings suggest that both activated and inhibitory T cell populations are expanded by DC/breast carcinoma fusions. (*See* Figure 9B). Of note, fusion stimulation of allogeneic T cells resulted in a similar increase in CD4+25+69+ T cells (5 fold) but a significantly greater expansion of GITR (25 fold) and CTLA-4 (15 fold) positive populations. (*See* Figure 9C).

The profile of cytokine expression in the CD4+CD25+ T cell population following stimulation with DC/breast carcinoma fusions using intracellular flow cytometric analysis was also examined. In 14 successive studies, the mean percentage of CD4+CD25+ T cells expressing IFNγ was 40% (± 6.9 SEM) and 68% (± 6.1 SEM) prior to and following fusion cell stimulation (p=0.005), respectively. (*See* Figure 10A and 10B). Similarly, the percentage of CD4+CD25+ T cells expressing the inhibitory cytokine, IL-10 (*see* Figure 10B) rose from 20% (± 4.9 SEM) to 59% (± 8.4 SEM) (p=0.0002).

Finally, the impact of fusion cell stimulation on the intracellular expression of Foxp3, a marker considered to be specific for regulatory T cells was assessed. Foxp3 expression increased from 26.5% (± 5.4 SEM; n=9) to 63% (± 10.6 SEM; n=9) (p=0.01) of the unstimulated and fusion stimulated CD4+CD25+ T cell populations respectively. (*See* Figure 10B). As such, fusion cells induce the expansion of both immunostimulatory and immunosuppressive elements resulting in a complex response in which regulatory T cells may prevent the development of sustained effective anti-tumor immunity.

### Example 5: Effects of exogenous IL-12, IL-18, and CpG ODN (TLR 9 agonist) on the fusion mediated stimulation of autologous T cells

The addition of IL-12, IL-18, and the TLR agonists, imidazoquinolone (TLR 7/8) and CPG-ODN (TLR 9) to the coculture of DC/breast carcinoma fusions and autologous T cells was examined to determine whether there was any increase in the prevalence of activated T cells following addition of the secondary stimulatory molecule. DC/breast carcinoma fusions were cocultured for 5-7 days with autologous T cells in the presence or absence of IL-12 (10 ng/ml; R & D Systems), IL-18 (10 ng/ml), or CPG ODN (10 µg/ml, Coley Pharmaceutical Group, Ottawa, Canada). The CpG ODN 2395 consisted of a hexameric CpG motif, 5'-TCGTCGTTTT-3' (SEQ ID NO:2), linked by a T spacer to the GC-rich palindrome sequence 5'-CGGCGCGCGCCG-3' (SEQ ID NO:3). A control CpG ODN without stimulatory sequences was simultaneously tested in each experiment. Regulatory and activated T cell populations were quantified as outlined above.

### Effect of TLR agonists on DC maturation and fusion mediated stimulation of T cell populations

In an effort to bias the T cell response towards an activated phenotype and limit the influence of regulatory T cells, the effect of the TLR 9 agonist, CPG ODN on vaccine response. TLR agonists activate elements of the innate immune response and have been shown to augment vaccine efficacy was studied. Specifically, the capacity of CPG ODN to modulate fusion mediated stimulation of activated and inhibitory T cell populations by quantifying expression of IFNγ as compared to IL-10 and Foxp3 in CD4/CD25+ cells was examined. Additionally, the effect of adding the stimulatory cytokines IL-12 and IL-18 on the phenotypic profile of T cells cocultured with DC/breast carcinoma fusions was also assessed. A 2.5 fold increase was seen in the fusion stimulated CD4+CD25+ T cells in the presence of CpG ODN and IL-18, respectively (p=0.0004 and p=0.006). In contrast no significant increase in CD4/CD25+ cells was seen when IL-12 was added to the cocultures of T cells and DC/breast cancer fusions. (*See* Figure 11A).

The addition of CPG, IL-12 or IL-18 decreased the percentage of CD4/CD25+ manifesting the phenotypic characteristics of regulatory T cells as manifested by Foxp3 expression (p=0.024, p=0.042, p= 0.016, respectively). In concert with these findings, expression of IL-10 in the CD4+CD25+ T cells was significantly lowered in cocultures pulsed with the addition of CpG ODN (19.8% ±4.1, n=7; p=0.002) and IL-18 (18.3% ±5.1, n=4; p=0.0004) as compared to T cells stimulated by DC/breast carcinoma fusions alone (59.3% ±8.4, n=14). (*See* Figure 11B). Of note, a decrease in the mean percentage of CD4+CD25+ T cells expressing IFNγ and IL-10 was also seen following the addition of CpG and IL-18 to the coculture of fusions and autologous T cells. (*See* Figure 11C). These results demonstrate that the addition of IL-12 or TLR agonists potentially enhances vaccine efficacy by limiting the presence of immunosuppressive regulatory cells.

### Example 6: Effect of anti-CD3/CD28 stimulation of T cells on DC/breast carcinoma fusion cell responses

As another strategy to bias the vaccine response toward immune activation, the effect of antibody mediated ligation of CD3 and CD28 on response to the DC/breast carcinoma fusion vaccine was examined. Anti-CD3/CD28 provides an antigen independent stimulus resulting in the expansion of activated or inhibitory T cells, dependent on the nature of the surrounding immunologic milieu. Thus, it was hypothesized that sequential stimulation with DC/breast carcinoma fusions followed by anti-CD3/CD28 would amplify the response of T cells that had been primarily activated by the fusion vaccine.

T cells were activated for 48h by exposure to the immobilized monoclonal antibodies, anti-CD3 (clone-UCHT1; Pharmingen) and anti-CD28 (clone-CD28.2; Pharmingen; CD3i/CD28i). Twenty-four-well non-tissue culture-treated plates (Falcon, Fisher) were coated with each of the antibodies (1 ug/ml in PBS) at 0.5 ml/well and left overnight at 4°C. The plates were blocked with 1% BSA and T cell preparations were loaded onto them at a density of 2x10⁶ cells/well. T cells were stimulated with anti-CD3/CD28 (48 hours) or DC/breast carcinoma fusions alone (5-7 days), fusions followed by exposure to anti-CD3/CD28, or anti-CD3/CD28 followed by fusion cells. T cells were harvested and proliferation was determined by uptake of tritiated thymidine. T cells binding the MUC1 tetramer were quantified. The percentage of T cells expressing markers consistent with a regulatory (Foxp3) and activated (CD69, IFNγ) phenotype were quantified.

In serial studies, limited proliferation of T cells was observed following exposure to CD3/CD28 alone (SI 1.6) or DC/breast carcinoma fusions (SI 3.1). (*See* Figure 12A) In contrast, a marked increase in T cell expansion was noted when T cells were first stimulated with DC/breast carcinoma fusions and then expanded with by anti-CD3/CD28 (SI 25.9). Of note, no increase in proliferation was observed when T cells were first exposed to anti-CD3/CD28 and then cultured with DC/breast carcinoma fusions (SI 1.5). Sequential stimulation with DC/breast carcinoma fusion and anti-CD3/CD28 resulted in the specific expansion of tumor reactive T cells. In three serial studies, exposure to anti-CD3/CD28 following fusion cell stimulation induced a 13.7 mean fold increase in MUC1 tetramer binding cells. (*See* Figure 12B). In contrast, the percentage of MUC1 tetramer + cells remained at baseline levels following stimulation with anti-CD3/CD28 alone.

Subsequently, the T cell phenotype of the expanded population was assessed. The percentage of T cells expressing CD4/CD25 was markedly increased following sequential stimulation with DC/RCC fusions and anti CD3/CD28 (28%) as compared to T cell stimulated by anti-CD3/CD28 (11%) or fusions alone (10%). (*See* Figure 12C). The addition of anti CD3/CD28 resulted in an approximately 5 fold increase in the percent of cells that coexpressed CD4, CD25, and CD69 consistent with an activated phenotype (Figure 12D). Similarly, a 4- and 3-fold increase in the percentage of cells that expressed IFNγ, respectively, was observed with sequential stimulation with fusions and anti CD3/CD28 as compared to fusion or anti-CD3/CD28, respectively. In contrast, an approximately 5 fold increase of regulatory T cells was also observed as manifested by an increase in CD4/CD25+ T cells that expressed Foxp3. (*See* Figure 12D).

These data suggest that fusion mediated stimulation followed by anti-CD3/CD28 expansion resulted in increased levels of both activated and regulatory T cells.

### Example: 7 Vaccination of Patients with Metastatic Breast Cancer with Dendritic Cell/Breast Cancer Fusions in Conjunction with IL-12

In order to study the safety, immunologic response, and clinical effect of vaccination with the dendritic cell (DC)/breast cancer fusions, the fusions are administered in conjunction with IL-12 in patients with metastatic breast cancer. DC/breast carcinoma fusion cells present a broad array of tumor associated antigens in the context of DC mediated costimulation. Fusion cells stimulate tumor specific immunity with the capacity to lyse autologous tumor cells. In clinical studies, vaccination with fusion cells was well tolerated, induced immunologic responses in a majority of patients, and results in disease regression in subset of patients. Administration of the vaccine in conjunction with IL-12 was hypothesized to further enhance vaccine response by promoting T cell activation.

The nature of DC/breast carcinoma fusions with respect to their phenotypic characteristics as antigen presenting cells and their capacity to stimulate anti-tumor immunity was examined. DC/breast carcinoma fusions strongly expressed costimulatory, adhesion, and maturation markers as well as the stimulatory cytokines, IL-12 and IFNγ. In addition, fusion cells expressed CCR7 necessary for the migration of cells to sites of T cell traffic in the draining lymph nodes. In keeping with these findings, fusions generated with immature and mature DCs potently stimulated CTL mediated lysis of autologous tumor targets.

Subsequently, the nature of the T cell response to DC/'breast carcinoma fusions with respect to the presence of activated and regulatory T cells was examined. DC/breast carcinoma fusions stimulated a mixed population of cells characterized by CD4/CD25/CD69 and CD4/CD25/Foxp3+ cells. The increased presence of regulatory cells was thought to potentially inhibit the in vivo efficacy of the fusion cell vaccine. As such, several strategies were examined to bias the fusion mediated T cell response towards activated cells. Addition of IL-12, TLR7/8 agonists, CPG ODN, or IL-18 increased the relative presence of activated as compared to regulatory cells.

To further define the nature of the T cell response to DC/breast carcinoma fusions, the functional characteristics of the expanded T cell population that co-express CD4 and CD25 were examined. Following stimulation with fusion cells, increased presence of CD4/CD25/FOXP3 cells are noted with mean levels of 26% and 63% of total CD4/CD25 cells observed prior to and following fusion coculture, respectively. CD4/CD25high cells that uniformly express FOXP3 were isolated by FACS sorting and analyzed for their capacity to inhibit mitogen and antigen specific responses of CD4/CD25- cells. CD4/CD25- T cells were cultured with PHA (2µg/ml) or anti-CD3 for 3 days in the presence or absence of CD4/CD25^{high} cells at a 1:1 ratio. Presence of the CD4/CD25^{high} cells resulted in significant inhibition of proliferation as determined by thymidine uptake following overnight pulsing. Similarly, peripheral blood mononuclear cells were cultured with tetanus toxoid (10µg/ml) for 5 days in the presence or absence of CD4/CD25^{high} cells at a 1:1 ratio. Presence of the CD4/CD25^{high} cells resulted in significant inhibition of T cell response to tetanus as determined by the stimulation index (thymidine uptake of PBMC and tetanus toxoid/thymidine uptake of PBMC alone).

Foxp3 expression was confirmed on the CD4/CD25^{high} cells sorted cells by FACS and immunocytochemical analyses. These data demonstrate that DC/breast carcinoma fusions induce the expansion of a T cell population with phenotypic and functional characteristics of regulatory T cells

### Selective expansion of activated T cells with DClbreast carcinoma fusions followed by anti-CD3/CD28

Several strategies were examined to enhance the capacity of DC/breast carcinoma fusions to stimulate anti-tumor immunity and limit the expansion of regulatory T cells. It was hypothesized that the combination of antigen specific stimulation with DC/tumor fusions and nonspecific ligation of the T cell costimulatory complex (CD3/CD28) would result in the activation of tumor specific lymphocytes. It was demonstrated that combined stimulation with DC/breast carcinoma fusion and anti-CD3/CD28 resulted in the expansion of tumor reactive T cells with a predominantly activated phenotype.

The phenotypic and functional characteristics of T cells undergoing sequential stimulation with DC/breast carcinoma fusions and anti-CD3/CD28 were examined (Figure 15). Limited proliferation of T cells was observed following exposure to anti-CD3/CD28 alone (SI: 1.5 ±0.5 SEM; n=7) or DC/breast carcinoma fusions (SI 3.1 ±1.2 SEM; n=7). However, a marked increase in T cell expansion was noted when T cells were first stimulated with DC/breast carcinoma fusions and then expanded with anti-CD3/CD28 (SI: 23 ±8.73 SEM; n=7). Of note, no increase in proliferation was observed when T cells were first exposed to anti-CD3/CD28 and then cultured with DC/breast carcinoma fusions (SI: 1.6 ±0.3 SEM; n=6).

Sequential stimulation with DC/breast carcinoma fusion and anti-CD3/CD28 resulted in the specific expansion of tumor reactive T cells. Exposure to anti-CD3/CD28 following fusion cell stimulation induced a 13.7 mean fold increase in MUC1 tetramer binding cells (n=3). The percentage of MUC1 tetramer + cells remained at baseline levels following stimulation with anti-CD3/CD28 alone.

With regard to the phenotype of the expanded T cell population, the percentage of T cells expressing the CD4+CD25+ phenotype was markedly increased following sequential stimulation with DC/tumor fusions and anti-CD3/CD28 (28%) as compared to T cell stimulated by anti-CD3/CD28 (11%) or fusions alone (10%) (n=6). As compared to fusion cells alone, sequential stimulation with DC/breast carcinoma fusions and anti-CD3/CD28 resulted in a 5 and 4 fold increase of CD4+CD25+ cells that coexpressed CD69and IFNγ. In contrast, an approximately 5 fold increase of regulatory T cells was also observed as manifested by an increase in CD4+CD25+ T cells that expressed Foxp3. These results suggest that fusion mediated stimulation followed by anti-CD3/CD28 expansion induces increased levels of both activated and regulatory T cells.

Future clinical trials will involve vaccination of metastatic breast cancer patients with DC/breast carcinoma fusions in conjunction with IL-12.

### Example 8: Stimulation of autologous T cell proliferation by DC/multiple myeloma fusions

Similar findings were observed in experiments using autologous fusion and T cells derived from a patient with multiple myeloma (MM). DCs were generated from adherent mononuclear cells and fused with autologous myeloma cells using the methods described herein. Autologous T cells were isolated using a T cell separation column. T cells derived from a patient with multiple myeloma were cocultured with fusion cells for 7 days at a fusion to T cell ratio of 1:10, or cocultured with fusion cells for 5 days followed by anti-CD3/CD28 coated plates for 48 h. Following stimulation, T cell proliferation was measured by uptake of tritiated thymidine following an overnight pulse. Sequential stimulation with DC/myeloma fusions followed by anti-CD3/CD28 markedly increased the level of T cell proliferation as compared to T cells stimulated by fusion cells alone (Figure 13).

Sequential stimulation with DC/MM fusions and anti-CD3/CD28 resulted in increased levels of activated T cells as defined by CD4+/CD25+/CD69+ cells. As compared to cells stimulated by anti-CD3/CD28 alone, a 27 and 39 fold increase in the percent of CD4/25/CD69 cells (of the total population) was observed following stimulation with DC/MM fusions alone or sequential stimulation with DC/MM fusions and anti-CD3/CD28. Subsequently, the capacity of T cells stimulated by DC/MM fusions and anti-CD3/CD28 to lyse autologous MM targets was examined. Patient derived T cells were stimulated by autologous DC/MM fusions alone for 7 days or with DC/MM fusions for 5 days with the subsequent exposure to anti-CD3/CD28 for 48 hours. Lysis of autologous myeloma cells was measured in a standard chromium release assay. T cells stimulated by DC/MM fusions followed by anti-CD3/CD28 demonstrated high levels of CTL mediated lysis of autologous myeloma targets in excess to that observed with T cells stimulated by DC/MM fusions alone (Figure 14). These findings demonstrate that sequential stimulation with DC/MM fusions and anti-CD3/CD28 results in the selective expansion of activated tumor specific T cells with the capacity to lyse tumor targets. This approach thus offers an ideal platform for the adoptive immunotherapy for multiple myeloma.

### Equivalents

While this invention has been particularly shown and described with references to preferred embodiments thereof, it will be understood by those skilled in the art that various changes in form and details may be made therein without departing from the spirit and scope of the invention as defined by the appended claims. Those skilled in the art will recognize or be able to ascertain using no more than routine experimentation, many equivalents to the specific embodiments of the invention described specifically herein. Such equivalents are intended to be encompassed in the scope of the claims.

The invention may be further defined with reference to the following clauses:
1. A method of producing a substantially pure, educated, expanded, antigen-specific population of immune effector cells, wherein the immune effector cells are T-lymphocytes and wherein said population comprises CD4⁺ immune effector cells and cytotoxic CD8⁺ immune effector cells, the method comprising:
   a) providing a plurality of hybrid cells, each of which hybrid cells is generated by fusion between at least one dendritic cell and at least one tumor or cancer cell that expresses a cell-surface antigen, wherein the dendritic cell and the tumor or cancer cell are from the same species, wherein the dendritic cell can process and present antigens, and wherein at least half of the hybrid cells express, in an amount effective to stimulate the immune system, (a) MHC class II molecule, (b) B7, and (c) the cell-surface antigen;
   b) contacting a population of immune effector cells with the plurality of hybrid cells, thereby producing a population of educated, antigen-specific immune effector cells; and
   c) contacting said population of educated, antigen-specific immune effector cells with anti-CD3/CD28 antibody, wherein said contacting results in an increase in T cell expansion, T cell activity, or tumor-reactive T cells as compared to exposure to said hybrid cells alone, or to said anti-CD3/CD28 antibody alone, thereby producing the substantially pure, expanded, educated, antigen-specific population of immune effector cells.
2. The method of clause 1, wherein the method further comprises contacting the population with a compound that removes or decreases the activity of regulatory T cells following expansion.
3. The method of clause 2, wherein said compound is a cytokine.
4. The method of clause 1, wherein the methods further comprises the step of removing or decreasing the activity of regulatory T cells by the use of selection methods or by the silencing of key genes using siRNAs.
5. The method of clause 1, wherein contacting the population with said anti-CD3/CD28 antibody results in at least a two-fold increase in activated T cells.
6. The method of clause 1, wherein contacting the population with said anti-CD3/CD28 antibody results in at least a two-fold increase in tumor reactive T-cells.
7. The method of clause 1, wherein contacting the population with said anti-CD3/CD28 antibody results in at least a two-fold increase in T-cell expansion.
8. The method of clause 1, wherein the anti-CD3/CD28 antibody is bound to a flat substrate.
9. The method of clause 8, wherein the immune effector cells are expanded in at least 24 hours.
10. The method of clause 1, wherein the immune effector cells are genetically modified cells.
11. The method of clause 1, wherein the hybrid cells are genetically modified cells.
12. The method of clause 10, wherein the genetic modification comprises introduction of a polynucleotide.
13. The method of clause 12, wherein the polynucleotide encodes a peptide, a ribozyme, an antisense sequence, a hormone, an enzyme, a growth factor, or an interferon.
14. The method of clause 1, wherein the immune effector cells are naive prior to culturing with the hybrid cells.
15. The method of clause 1, wherein the immune effector cells are cultured with the hybrid cells in the presence of a cytokine or an adjuvant.
16. The method of clause 15, wherein the cytokine is IL-7, IL-12, IL-15, or IL-18.
17. The method of clause 15, wherein the adjuvant is CPG ODN, a TLR7/8 agonist, or a TLR3 agonist
18. The method of clause 1, wherein the expanded, educated, antigen-specific population of immune effector cells is maintained in a cell culture medium comprising a cytokine.
19. The method of clause 18, wherein the cytokine is IL-7.
20. The method of clause 1, wherein the dendritic cell and the tumor or cancer cell that expresses one or more antigens are autologous.
21. The method of clause 1, wherein the dendritic cell and the tumor or cancer cell that expresses one or more antigens are allogeneic.
22. The method of clause 1, wherein the dendritic cell is derived or mobilized from peripheral blood, bone marrow or skin.
23. The method of clause 1, wherein the dendritic cell is derived from a dendritic cell progenitor cell.
24. The method of clause 23, wherein the dendritic cell and the tumor or cancer cell are obtained from the same individual.
25. The method of clause 24, wherein the species is human.
26. The method of clause 23, wherein the dendritic cell and the tumor or cancer cell are obtained from different individuals of the same species.
27. The method of clause 26, wherein the species is *Homo sapiens.*
28. The method of clause 1, wherein said tumor or cancer cells are breast cancer cells, ovarian cancer cells, pancreatic cancer cells, prostate gland cancer cells, renal cancer cells, lung cancer cells, urothelial cancer cells, colon cancer cells, rectal cancer cells, or hematological cancer cells.
29. The method of clause 28, wherein said hematological cancer cells are selected from the group consisting of acute myeloid leukemia cells, acute lymphoid leukemia cells, multiple myeloma cells, and non-Hodgkin's lymphoma cells.
30. A substantially pure population comprising expanded, educated, antigen-specific immune effector cells, wherein said population comprises educated, antigen-specific immune effector cells, wherein said immune effector cells are educated by hybrid cells, wherein the hybrid cells comprise dendritic cells fused to tumor or cancer cells that express one or more antigens, wherein the dendritic cell and the tumor or cancer cell are from the same species, wherein the dendritic cell can process and present antigens, and wherein at least half of the fused cells express, in an amount effective to stimulate the immune system, (a) a MHC class II molecule, (b) B7, and (c) the cell-surface antigen, and wherein said educated, immune effector cells are expanded in culture in the presence of anti-CD3/CD28 antibody, wherein following expansion in culture in the presence of anti-CD3/CD28 antibody, T cell expansion in the population is at least two-fold increased as compared to immune effector cells exposed to said hybrid cells alone, T-cell activation in the population is at least two fold increased as compared to immune effector cells exposed to said hybrid cells alone, tumor-reactive T-cells in the population are at least two fold increased as compared to immune effector cells exposed to said hybrid cells alone, or any combination thereof.
31. The population of clause 30, wherein the dendritic cell and the tumor or cancer cell are obtained from the same individual.
32. The population of clause 31, wherein the species is human.
33. The population of clause 30, wherein the dendritic cell and the tumor or cancer cell are obtained from different individuals of the same species.
34. The population of clause 33, wherein the species is *Homo sapiens.*
35. The population of clause 30, wherein, when said tumor or cancer cell is a renal carcinoma cell, T cell proliferation in the population is at least about two-fold increased as compared to immune effector cells exposed to said hybrid cells alone, following expansion in culture in the presence of anti-CD3/CD28 antibody.
36. The population of clause 30, wherein, when said tumor or cancer cell is a renal carcinoma cell, the presence of memory effector cells in the population is increased at least about two fold as compared to immune effector cells exposed to said hybrid cells alone, following expansion in culture in the presence of anti-CD3/CD28 antibody.
37. The population of clause 30, wherein, when said tumor or cancer cell is a renal carcinoma cell, T cell activation in the population is at least about two-fold increased as compared to immune effector cells exposed to said hybrid cells alone, following expansion in culture in the presence of anti-CD3/CD28 antibody.
38. The population of clause 30, wherein, when said tumor or cancer cell is a renal carcinoma cell, the presence of cells expressing IFNγ and granzyme B in the population is increased as compared to immune effector cells exposed to said hybrid cells alone, following expansion in culture in the presence of anti-CD3/CD28 antibody.
39. The population of clause 38, wherein the presence of cells expressing IFNγ in the population is increased at least about two-fold as compared to immune effector cells exposed to said hybrid cells alone, following expansion in culture in the presence of anti-CD3/CD28 antibody.
40. The population of clause 38, wherein the presence of cells expressing wherein granzyme B in the population is increased at least about two-fold as compared to immune effector cells exposed to said hybrid cells alone, following expansion in culture in the presence of anti-CD3/CD28 antibody.
41. The population of clause 30, wherein, when said tumor or cancer cell is a renal carcinoma cell, tumor-reactive T cells in the population are at least about twofold increased as compared to immune effector cells exposed to said hybrid cells alone, following expansion in culture in the presence of anti-CD3/CD28 antibody.
42. A vaccine comprising the population of expanded, educated, antigen-specific immune effector cells of clause 30.
43. The vaccine of clause 42, further comprising a pharmaceutically acceptable carrier.
44 A method of treating cancer in an individual comprising administering the population of clause 30 to the individual, wherein an immune response is induced, and wherein said cancer is selected from the group consisting of breast cancer, ovarian cancer, pancreatic cancer, prostate gland cancer, renal cancer, lung cancer, urothelial cancer, colon cancer, rectal cancer, glioma, or hematological cancer.
45. The method of clause 44, wherein said hematological cancer is selected from the group consisting of acute myeloid leukemia, acute lymphoid leukemia, multiple myeloma, and non-Hodgkin's lymphoma.
46. The method of clause 44, wherein said cancer is breast cancer.
47. The method of clause 44, wherein the dendritic cell and the tumor or cancer cell are obtained from the same individual.
48. The method of clause 47, wherein the species is human.
49. The method of clause 44, wherein the dendritic cell and the tumor or cancer cell are obtained from different individuals of the same species.
50. The method of clause 49, wherein the species is *Homo sapiens.*
51. The method of clause 44 further comprising co-administering an effective amount of a plurality of hybrid cells, each of which hybrid cells is generated by fusion between at least one dendritic cell and at least one tumor or cancer cell that expresses a cell-surface antigen, wherein the dendritic cell and the tumor or cancer cells are from the same species, and wherein at least half of the hybrid cells express, in an amount effective to stimulate the immune system, (a) MHC class II molecule, (b) B7, and (c) the cell-surface antigen.
52. The method of clause 51, wherein said co-administration occurs sequentially.
53. The method of clause 51, wherein said co-administration occurs simultaneously.
54. The method of clause 44, wherein the individual is administered a treatment to deplete lymphocytes prior to administration of said population.
55. The method of clause 54, wherein said treatment induces lymphopenia in said individual.
56. The method of clause 55, wherein said treatment comprises administration of fludarabine or radiation.
57. The method of clause 44, wherein said cells are administered to said individual subsequent to stem cell transplantation.
58. A method of testing a peptide for antigenic activity, the method comprising:
   (a) providing a hybrid cell comprising a fusion product of a dendritic cell and a tumor or cancer cell, wherein said hybrid cell expresses B7 on its surface;
   (b) contacting the hybrid cell with an immune effector cell, thereby producing an educated immune effector cell;
   (c) contacting said educated immune effector cell with anti-CD3/CD28 antibody; and
   (d) contacting a target cell with said educated immune effector cell in the presence of a peptide, wherein lysis of said target cell identifies the peptide as an antigenic peptide.
59. A method of testing a peptide for antigenic activity, the method comprising:
   (a) providing a plurality of cells, wherein at least 5% of the cells of said plurality of cells are fused cells generated by fusion between at least one dendritic cell and at least one tumor or cancer cell that expresses a cell-surface antigen, wherein said fused cells express, in amounts effective to stimulate an immune response, (a) MHC class II molecule, (ii) B7, and (iii) the cell-surface antigen,
   (b) contacting a population of human T lymphocytes with the plurality of cells, wherein the contacting causes differentiation of effector cell precursor cells in the population of T lymphocytes to effector cells comprising cytotoxic T lymphocytes;
   (c) contacting said effector cells comprising cytotoxic T lymphocytes with anti-CD3/CD28 antibody; and
   (d) contacting a plurality of target cells with said effector cells comprising T lymphocytes in the presence of the peptide; wherein lysis of the plurality of target cells or a portion thereof identifies the peptide as an antigenic peptide that is recognized by the cytotoxic T lymphocytes.
60. A vaccine comprising a peptide identified according to the method of clause 58 and a carrier.
61. A vaccine comprising a peptide identified according to the method of clause 59 and a carrier.

## Claims

1. A method of producing a substantially pure, educated, expanded, antigen-specific population of immune effector cells, wherein the immune effector cells are T-lymphocytes and wherein said population comprises CD4⁺ immune effector cells and cytotoxic CD8⁺ immune effector cells, the method comprising:
a) providing a plurality of hybrid cells, each of which hybrid cells is generated by fusion between at least one dendritic cell and at least one tumor or cancer cell that expresses a cell-surface antigen, wherein the dendritic cell and the tumor or cancer cell are from the same species, wherein the dendritic cell can process and present antigens, and wherein at least half of the hybrid cells express, in an amount effective to stimulate the immune system, (a) MHC class II molecule, (b) B7, and (c) the cell-surface antigen;
b) contacting a population of immune effector cells with the plurality of hybrid cells, thereby producing a population of educated, antigen-specific immune effector cells; and
c) contacting said population of educated, antigen-specific immune effector cells with anti-CD3/CD28 antibody, wherein said contacting results in an increase in T cell expansion, T cell activity, or tumor-reactive T cells as compared to exposure to said hybrid cells alone, or to said anti-CD3/CD28 antibody alone, thereby producing the substantially pure, expanded, educated, antigen-specific population of immune effector cells.

2. A substantially pure population comprising expanded, educated, antigen-specific immune effector cells, wherein said population comprises educated, antigen-specific immune effector cells, wherein said immune effector cells are educated by hybrid cells, wherein the hybrid cells comprise dendritic cells fused to tumor or cancer cells that express one or more antigens, wherein the dendritic cell and the tumor or cancer cell are from the same species, wherein the dendritic cell can process and present antigens, and wherein at least half of the fused cells express, in an amount effective to stimulate the immune system, (a) a MHC class II molecule, (b) B7, and (c) the cell-surface antigen, and wherein said educated, immune effector cells are expanded in culture in the presence of anti-CD3/CD28 antibody, wherein following expansion in culture in the presence of anti-CD3/CD28 antibody, T cell expansion in the population is at least two-fold increased as compared to immune effector cells exposed to said hybrid cells alone, T-cell activation in the population is at least two fold increased as compared to immune effector cells exposed to said hybrid cells alone, tumor-reactive T-cells in the population are at least two fold increased as compared to immune effector cells exposed to said hybrid cells alone, or any combination thereof.

3. A vaccine comprising the population of expanded, educated, antigen-specific immune effector cells of claim 2.

4. A method of treating cancer in an individual comprising administering the population of claim 30 to the individual, wherein an immune response is induced, and wherein said cancer is selected from the group consisting of breast cancer, ovarian cancer, pancreatic cancer, prostate gland cancer, renal cancer, lung cancer, urothelial cancer, colon cancer, rectal cancer, glioma, or hematological cancer.

5. A method of testing a peptide for antigenic activity, the method comprising:
(a) providing a hybrid cell comprising a fusion product of a dendritic cell and a tumor or cancer cell, wherein said hybrid cell expresses B7 on its surface;
(b) contacting the hybrid cell with an immune effector cell, thereby producing an educated immune effector cell;
(c) contacting said educated immune effector cell with anti-CD3/CD28 antibody; and
(d) contacting a target cell with said educated immune effector cell in the presence of a peptide, wherein lysis of said target cell identifies the peptide as an antigenic peptide.

6. A method of testing a peptide for antigenic activity, the method comprising:
(a) providing a plurality of cells, wherein at least 5% of the cells of said plurality of cells are fused cells generated by fusion between at least one dendritic cell and at least one tumor or cancer cell that expresses a cell-surface antigen, wherein said fused cells express, in amounts effective to stimulate an immune response, (a) MHC class II molecule, (ii) B7, and (iii) the cell-surface antigen,
(b) contacting a population of human T lymphocytes with the plurality of cells, wherein the contacting causes differentiation of effector cell precursor cells in the population of T lymphocytes to effector cells comprising cytotoxic T lymphocytes;
(c) contacting said effector cells comprising cytotoxic T lymphocytes with anti-CD3/CD28 antibody; and
(d) contacting a plurality of target cells with said effector cells comprising T lymphocytes in the presence of the peptide; wherein lysis of the plurality of target cells or a portion thereof identifies the peptide as an antigenic peptide that is recognized by the cytotoxic T lymphocytes.

7. A vaccine comprising a peptide identified according to the method of claim 5 and a carrier.

8. A vaccine comprising a peptide identified according to the method of claim 6 and a carrier.


SEQUENCE LISTING

<110>  Dana-Farber Cancer Institute, Inc.
       Beth Israel Deaconess Medical Center
       Avigan, David
       Kufe, Donald

<120>  Stimulation of Anti-Tumor Immunity Using Dendritic Cell/Tumor
       Cell Fusions and Anti-CD3/CD28

<130>  20363-047001WO

<140>  PCT/US2008/082750
<141>  2008-11-07

<150>  61/002,538
<151>  2007-11-08

<160>  3

<170>  PatentIn version 3.4

<210>  1
<211>  9
<212>  PRT
<213>  Artificial sequence

<220>
<223>  MUC1-specific epitopes M1.2 (MUC112-20)

<400>  1

Leu Leu Leu Leu Thr Val Leu Thr Val
1               5

<210>  2
<211>  10
<212>  DNA
<213>  Artificial sequence

<220>
<223>  hexameric CpG motif

<400>  2
tcgtcgtttt                                                              10

<210>  3
<211>  12
<212>  DNA
<213>  Artificial sequence

<220>
<223>  GC-rich palindrome sequence

<400>  3

cggcgcgcgc cg 12